# EUROPEAN PATENT SPECIFICATION

(11) **EP 3 693 362 B1**
(45) Date of publication and mention of the grant of the patent: **20.12.2023**
(21) Application number: 18864191.4
(22) Date of filing: 02.10.2018
(51) Int. Cl.: C07D 307/60, A01H 3/04, A01N 43/40, A01N 47/16, A01P 21/00, C07D 405/12, C07D 413/12

(54) **PARASITIC PLANT GERMINATION REGULATOR**
KEIMUNGSREGULATOR FÜR PARASITISCHE PFLANZEN
MODIFICATEUR DE GERMINATION DE PLANTES PARASITES

(30) Priority: 03.10.2017 JP 2017193773
(43) Date of publication of application: 12.08.2020
(73) Proprietor: National University Corporation Tokai National Higher Education and Research System, Nagoya-shi, Aichi 464-8601 (JP)
(72) Inventor: TSUCHIYA, Yuichiro, Nagoya-shi Aichi 464-8601 (JP); URAGUCHI, Daisuke, Nagoya-shi Aichi 464-8601 (JP); OOI, Takashi, Nagoya-shi Aichi 464-8601 (JP); KINOSHITA, Toshinori, Nagoya-shi Aichi 464-8601 (JP); MOCHIDA, Keiko, Nagoya-shi Aichi 464-8601 (JP)
(74) Representative: Vossius & Partner Patentanwälte Rechtsanwälte mbB
(86) International application number: PCT/JP2018/036785
(87) International publication number: WO 2019/069889

(56) References cited:
- EP-A1- 2 562 171
- EP-A1- 2 623 502
- WO-A1-2013/140946
- WO-A1-2013/140946
- WO-A1-2013/174925
- WO-A1-2017/002898
- WO-A1-2017/002898
- JP-A- 2006 282 513
- US-A1- 2013 085 068
- YONEYAMA, KOICHI et al.: "Chemical stimulation of seed germination of root parasitic weeds, Striga and Orobanche spp.", Chemical Regulation of Plants, vol. 34, no. 2, 1999, pages 181-190, XP055591814,
- SUGIMOTO, YUKIHIRO et al.: "Germination stimulants for the seeds of root parasitic plants", Root Research, vol. 12, no. 2, 2003, pages 51-56, XP009520263, DOI: 10.3117/rootres.12.51

## Description

### Technical Field

The present invention relates to a compound useful as a parasitic plant germination regulator, and to a parasitic plant germination regulator.

### Background Art

Parasitic plants represented by plants belonging to the genus *Striga* (in the present specification, sometimes abbreviated as "*Striga*") parasitize major crops, such as corn, rice, and legumes, and cause poor growth. Its damage has spread to Africa, Asia, Australia, the United States, etc. In Africa, in particular, the damage is said to cost 10 billion US dollars per year.

Parasitic plant seeds can survive in soil for a long period of time, detect germination stimulants (e.g., strigolactone) secreted from the roots of surrounding host crops, thus undergoing germination, and parasitize the roots of the host crops. The seeds from the plant body of parasitic plants that grow on the ground also contaminate the surrounding soil. Accordingly, once soil is contaminated with the seeds of parasitic plants, the soil will continue to be damaged by the parasitic plants, and the range of soil contaminated with the seeds will also gradually expand if no measures are taken.

To address issues involving parasitic plants, it is said to be effective to apply a parasitic plant germination regulator to soil contaminated with parasitic plant seeds. For example, if a parasitic plant germination inducer is used in an environment where no host plant is present, parasitic plants after germination cannot parasitize a host plant, and will wither and die, allowing the soil to be purified. Therefore, the development of substances that more efficiently induce the germination of parasitic plants has been in demand.

Strigolactones and their derivatives are disclosed, for example, in Non-patent Literature (NPL) 1. Strigolactones are carotenoid derivatives. As shown below 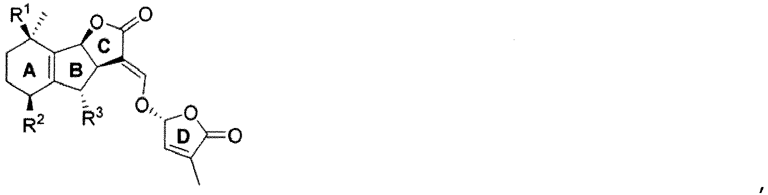 strigolactones are characterized by a complex basic skeleton in which a tricyclic terpenoid containing a lactone ring (ring ABC) is further connected to another lactone ring (ring D) via an enol ether, and contain many asymmetric centers. Further, strigolactones and their derivatives are known to exist in root exudates of many plants and exert phytohormone actions, such as branch growth inhibition (NPL 2).

Compounds having a basic skeleton different from that of strigolactones or their derivatives are disclosed in, for example, Patent Literature (PTL) 1. The carbamate compound disclosed in PTL 1 can be obtained by producing carbamate from 3-methylbutenolide. However, to exert the germination-stimulating activity disclosed in PTL 1, a concentration as high as 10 µM is required, and the activity is assumed to remain lower than that of strigolactones or their derivatives.

### Citation List

### Patent Literature

PTL 1: WO 2011/125714
PTL 2: WO 2017/002898

### Non-patent Literature

NPL 1: Plant Cell Physiol. 51(7): 1095-1103 (2010)
NPL 2: Nature 455, 189-194 (2008)

### Summary of Invention

### Technical Problem

The present inventors have reported that a compound that has a different basic skeleton from those of strigolactones or their derivatives has *Striga* germination regulation activity (PTL 2). In the course of conducting further extensive research, the inventors found that there was room for further improvement in terms of germination-inducing activity, synthesis costs, and the like.

An object of the present invention is to provide a compound with parasitic plant germination regulation activity (in particular, parasitic plant germination-inducing activity), and a parasitic plant germination regulator comprising the compound.

### Solution to Problem

As a result of extensive research in view of the problems, the present inventors etc. have found that compounds represented by Formula (1) exhibit parasitic plant germination regulation activity (in particular, parasitic plant germination-inducing activity).

More specifically, the present invention encompasses the following embodiments.

Item 1. A compound represented by Formula (1): 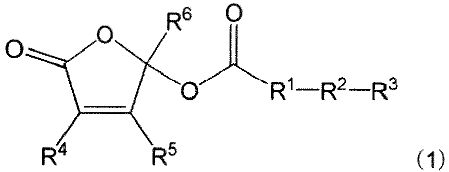 as defined in claim 1, an agriculturally acceptable salt thereof, a hydrate thereof, or a solvate thereof.

### (to be continued on page 7)

Item 2. A parasitic plant germination regulator comprising a compound represented by Formula (1), an agriculturally acceptable salt thereof, a hydrate thereof, or a solvate thereof: 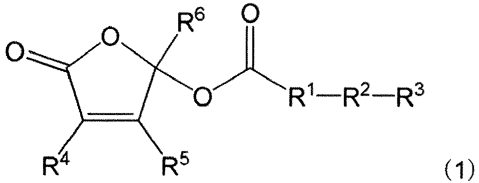 according to claim 4.

Item 3. A method for regulating parasitic plant germination, comprising applying the parasitic plant germination regulator any according to item 2 to soil containing parasitic plant seeds.

Item 4. Use of the compound according to item 1, an agriculturally acceptable salt thereof, a hydrate thereof, or a solvate thereof, for regulating parasitic plant germination, comprising applying the compound according to the invention to soil containing parasitic plant seeds.

### Advantageous Effects of Invention

The present invention provides a compound with parasitic plant germination regulation activity (in particular, parasitic plant germination-inducing activity) and a parasitic plant germination regulator comprising the compound. According to the present invention or according to one embodiment of the present invention, it is possible to provide a compound and parasitic plant germination regulator with higher parasitic plant germination regulation activity (in particular, parasitic plant germination-inducing activity). Further, according to the present invention or according to one embodiment of the present invention, it is possible to exhibit higher parasitic plant germination regulation activity (in particular, parasitic plant germination-inducing activity) while causing less effect on plants other than parasitic plants (e.g., crops in soil that contains parasitic plant seeds). Furthermore, according to the present invention or according to one embodiment of present invention, it is also possible to provide, at a lower cost, a compound with parasitic plant germination regulatory activity (in particular, parasitic plant germination-inducing activity) and a parasitic plant germination regulator comprising the compound. Therefore, the use of the present invention is practical to address damage caused by *Striga* widely all over the world, in particular, in countries and regions where damage caused by *Striga* is severe and yet no sufficient measures have been taken due to economic issues (in particular, Africa etc.).

### Description of Embodiments

In this specification, the expressions "comprising," "containing," and "including" include the concepts of "containing," "including," "consisting essentially of," and "consisting of."

### 1. Compound

In one embodiment, the present invention relates to a compound represented by Formula (1), an agriculturally acceptable salt thereof, a hydrate thereof, or a solvate thereof: 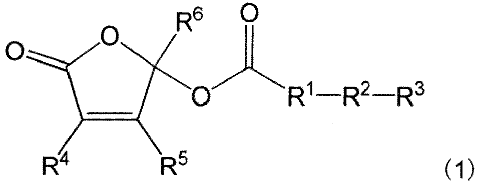 as defined in claim 1.

The details are as follows.

### 1-1. R¹

In Formula (1), R¹ is preferably a divalent group represented by Formula (Y): 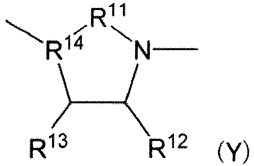 wherein
R¹¹ represents -(CHR^{11a}-)ₙ, wherein n represents an integer of 1 to 3, R^{11a}s are identical or different, and each represents hydrogen or alkyl, and when n is 2 or 3, two R^{11a}s, taken together with the adjacent carbon atoms, may form a ring,
R¹² and R¹³ are identical or different, and each represents hydrogen or alkyl, and R¹² and R¹³, taken together with the adjacent carbon atoms, may form a ring, and
R¹⁴ represents carbon or nitrogen.

In Formula (Y), n is preferably 2 or 3, and more preferably 2.

In Formula (Y), the alkyl represented by R^{11a} encompasses linear, branched, and cyclic (preferably linear or branched, and more preferably linear) alkyl groups. The carbon number of the alkyl is not particularly limited, and is, for example, 1 to 8, preferably 1 to 6, more preferably 1 to 4, even more preferably 1 to 2, and still more preferably 1. Specific examples of the alkyl include methyl, ethyl, n-propyl, isopropyl, n-butyl, isobutyl, tert-butyl, sec-butyl, n-pentyl, neopentyl, n-hexyl, 3-methylpentyl, and the like.

In Formula (Y), when n is 1, R^{11a} is preferably hydrogen. When n is 2 or 3, it is preferable that R^{11a}s be identical or different, and each represent hydrogen or alkyl; and it is more preferable that R^{11a}s be all hydrogen atoms.

In Formula (Y), the alkyl represented by R¹² or R¹³ encompasses linear, branched, and cyclic (preferably linear or branched, and more preferably linear) alkyl groups. The carbon number of the alkyl is not particularly limited, and is, for example, 1 to 8, preferably 1 to 6, more preferably 1 to 4, even more preferably 1 to 2, and still more preferably 1. Specific examples of the alkyl include methyl, ethyl, n-propyl, isopropyl, n-butyl, isobutyl, tert-butyl, sec-butyl, n-pentyl, neopentyl, n-hexyl, 3-methylpentyl, and the like.

The ring formed by two R^{11a}s taken together with the adjacent carbon atoms, and the ring formed by R¹² and R¹³ taken together with the adjacent carbon atoms are not particularly limited. Examples include aromatic rings, such as benzene ring; aliphatic rings, such as cyclohexane; heterocycles, such as pyrimidine, pyridine, piperidine, pyran, and thiophene; and the like.

In Formula (Y), R¹² and R¹³ are identical or different, and each represent hydrogen or alkyl; and it is preferable that R¹² and R¹³ both be hydrogen atoms.

In Formula (Y), R¹⁴ is preferably nitrogen.

In Formula (1), R¹ is preferably a divalent group represented by Formula (Y), and more preferably a divalent group represented by Formula (YA): 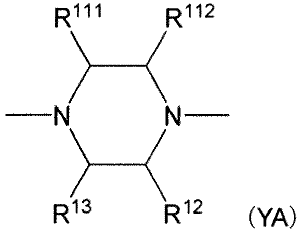 wherein
R¹² and R¹³ are as defined above, and
R¹¹¹ and R¹¹² are identical or different, and each represents hydrogen or alkyl, and R¹¹¹ and R¹¹², taken together with the adjacent carbon atoms, may form a ring.

In Formula (YA), the alkyl represented by R¹¹¹ or R¹¹² encompasses linear, branched, and cyclic (preferably linear or branched, and more preferably linear) alkyl groups. The carbon number of the alkyl is not particularly limited, and is, for example, 1 to 8, preferably 1 to 6, more preferably 1 to 4, even more preferably 1 to 2, and still more preferably 1. Specific examples of the alkyl include methyl, ethyl, n-propyl, isopropyl, n-butyl, isobutyl, tert-butyl, sec-butyl, n-pentyl, neopentyl, n-hexyl, 3-methylpentyl, and the like.

In Formula (YA), it is preferable that R¹¹¹ and R¹¹² be identical or different, and each represent hydrogen or alkyl; and it is more preferable that R¹¹¹ and R¹¹² be both hydrogen atoms.

In another preferred embodiment, R¹ in Formula (1) is a divalent group represented by Formula (Y'): 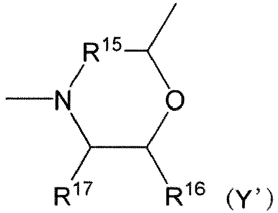 wherein
R¹⁵ represents a single bond or - (CHR^{15a}-)ₘ, wherein m represents 1 or 2, R^{15a}s are identical or different, and each represents hydrogen or alkyl, and when m is 2, two adjacent R^{15a}s, taken together with the adjacent carbon atom, may form a ring, and
R¹⁶ and R¹⁷ are identical or different, and each represents hydrogen or alkyl, and R¹⁶ and R¹⁷, taken together with the adjacent carbon atoms, may form a ring.

In Formula (Y'), R¹⁵ is preferably -(CHR^{15a}-)ₘ.

In Formula (Y'), m is preferably 1.

In Formula (Y'), the alkyl represented by R^{15a} is the same as the alkyl represented by R^{11a} mentioned above.

In Formula (Y'), the alkyl represented by R¹⁶ or R¹⁷ is the same as the alkyl represented by R¹² or R¹³ mentioned above.

### 1-2. R²

In Formula (1), R2 represents -S(O)₂-. 1-3. R³

In Formula (1), the aryl represented by R³ is optionally substituted phenyl.

In formula (1), the substituent that may be possessed by the aryl represented by R³ is not particularly limited. Examples include halogen, optionally substituted alkyl, optionally substituted alkoxy, optionally substituted alkylthio, optionally substituted alkenyl, optionally substituted alkynyl, optionally substituted amino, nitro, hydroxyl, cyano, -C(=O)R³⁰¹, -C(=O)OR³⁰², -C(=O)NR³⁰³R³⁰⁴, -S(=O)₁₋₂R³⁰⁵, -S(=O)₁₋₂NR³⁰⁶R³⁰⁷, and the like.

In formula (1), examples of halogen that may be possessed by the aryl represented by R³ include fluorine, chlorine, bromine, iodine, and the like.

In Formula (1), the optionally substituted alkyl that may be possessed by the aryl represented by R³ is not particularly limited. Examples include linear, branched, or cyclic (preferably linear or branched, and more preferably linear) alkyl groups containing 1 to 8, preferably 1 to 6, more preferably 1 to 5, even more preferably 2 to 5, and still more preferably 3 to 5 carbon atoms and being optionally substituted with halogen (e.g., fluorine, chlorine, bromine, iodine), oxo, hydroxyl, alkoxy, optionally substituted amino, phenyl, and the like. The number of substituents is not particularly limited and is, for example, 0 to 6, preferably 0 to 3, and more preferably 0. Examples of the optionally substituted alkyl include methyl, ethyl, n-propyl, isopropyl, n-butyl, isobutyl, sec-butyl, t-butyl, perfluoromethyl, perfluoroethyl, and the like.

In Formula (1), the optionally substituted alkoxy that may be possessed by the aryl represented by R³ is not particularly limited. Examples include linear or branched (preferably linear) alkoxy groups containing 1 to 8, preferably 1 to 6, more preferably 1 to 5, even more preferably 2 to 5, and still more preferably 3 to 5 carbon atoms, and being optionally substituted with halogen (e.g., fluorine, chlorine, bromine, iodine), oxo, hydroxyl, alkoxy, optionally substituted amino, phenyl, and the like. The number of substituents is not particularly limited and is, for example, 0 to 6, preferably 0 to 3, and more preferably 0. Examples of the optionally substituted alkoxy include methoxy, ethoxy, n-propoxy, isopropoxy, n-butoxy, isobutoxy, sec-butoxy, t-butoxy, perfluoromethoxy, perfluoroethoxy, and the like.

In Formula (1), the optionally substituted alkylthio that may be possessed by the aryl represented by R³ is not particularly limited. Examples include linear or branched (preferably linear) alkylthio groups containing 1 to 8, preferably 1 to 6, and more preferably 1 to 4 carbon atoms, and being optionally substituted with halogen (e.g., fluorine, chlorine, bromine, iodine), oxo, hydroxyl, alkoxy, optionally substituted amino, phenyl, and the like. The number of substituents is not particularly limited and is, for example, 0 to 6, and preferably 0 to 3. Examples of the optionally substituted alkylthio include methylthio, ethylthio, n-propylthio, isopropylthio, n-butylthio, isobutylthio, sec-butylthio, t-butylthio, perfluoromethylthio, perfluoroethylthio, and the like.

In Formula (1), the optionally substituted alkenyl that may be possessed by the aryl represented by R³ is not particularly limited. Examples include linear or branched (preferably linear) alkenyl groups containing 2 to 8, preferably 2 to 6, and more preferably 2 to 4 carbon atoms, and being optionally substituted with halogen (e.g., fluorine, chlorine, bromine, iodine), oxo, hydroxyl, alkoxy, optionally substituted amino, phenyl, and the like. The number of substituents is not particularly limited and is, for example, 0 to 6, and preferably 0 to 3. Examples of the optionally substituted alkenyl include vinyl, allyl, 1-propenyl, isopropenyl, butenyl, pentenyl, hexenyl, and the like.

In Formula (1), the optionally substituted alkynyl that may be possessed by the aryl represented by R³ is not particularly limited. Examples include linear or branched (preferably linear) alkynyl groups containing 2 to 8, preferably 2 to 6, and more preferably 2 to 4 carbon atoms, and being optionally substituted with halogen (e.g., fluorine, chlorine, bromine, iodine), oxo, hydroxyl, alkoxy, optionally substituted amino, phenyl, and the like. The number of substituents is not particularly limited and is, for example, 0 to 6, and preferably 0 to 3. Examples of the optionally substituted alkynyl include ethynyl, propynyl (e.g., 1-propynyl, 2-propynyl (propargyl)), butynyl, pentynyl, hexynyl, phenylacetylenyl, and the like.

In Formula (1), the optionally substituted amino that may be possessed by the aryl represented by R³ is not particularly limited. Examples include amino groups optionally substituted with linear or branched (preferably linear) alkyl and/or acyl containing 1 to 6, preferably 1 to 4, more preferably 1 to 2 carbon atoms. The number of alkyl and/or acyl substituents is preferably 1 to 2. (When the substituent(s) are alkyl and/or acyl, and the number of substituents is 2, the alkyl and/or acyl, taken together with the adjacent nitrogen atoms, may form a ring.) Examples of the optionally substituted amino include amino, methylamino, ethylamino, dimethylamino, diethylamino, ethylmethylamino, acetamide, and the like.

In formula (1), the substituents that may be possessed by the aryl represented by R³ is preferably halogen, optionally substituted alkyl, optionally substituted alkoxy, optionally substituted alkylthio, optionally substituted alkenyl, optionally substituted alkynyl, optionally substituted amino, nitro, and the like, more preferably, halogen, optionally substituted alkyl, optionally substituted alkoxy, nitro, and the like, and even more preferably optionally substituted alkyl, optionally substituted alkoxy, and the like.

R³⁰¹, R³⁰², R³⁰³, R³⁰⁴, R³⁰⁵, R³⁰⁶, and R³⁰⁷ each represent hydrogen, optionally substituted alkyl, or optionally substituted phenyl. The "optionally substituted alkyl" and "optionally substituted phenyl" here are as defined above for the substituents that may be possessed by the aryl represented by R³.

In Formula (1), the number of substituents of the aryl represented by R³ is, for example, 0 to 5, preferably 1 to 3, and more preferably 1.

In Formula (1), R³ is optionally substituted phenyl, and preferably a group represented by Formula (X): 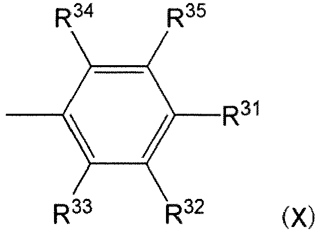 wherein R³¹, R³², R³³, R³⁴, and R³⁵ are identical or different, and each represents hydrogen, halogen, optionally substituted alkyl, optionally substituted alkoxy, optionally substituted alkylthio, optionally substituted alkenyl, optionally substituted alkynyl, optionally substituted amino, nitro, hydroxyl, cyano, -C(=O)R³⁰¹, -C(=O)OR³⁰², -C(=O)NR³⁰³R³⁰⁴, -S(=O)₁₋₂R³⁰⁵, -S(=O)₁₋₂, or NR³⁰⁶R³⁰⁷.

In Formula (X), various substituents represented by R³¹, R³², R³³, R³⁴, or R³⁵ are the same as those that may be possessed by the aryl represented by R³.

In Formula (X), it is preferable that one to three of R³¹, R³², R³³, R³⁴, and R³⁵ (preferably one of these, more preferably R³¹) be halogen, optionally substituted alkyl, optionally substituted alkoxy, or nitro, and the others be all hydrogen atoms, and it is more preferable that R³¹ be optionally substituted alkyl or optionally substituted alkoxy, and R³², R³³, R³⁴, and R³⁵ all be hydrogen atoms.

### 1-4. R⁴, R⁵ and R⁶

In Formula (1), examples of the halogen represented by R⁴, R⁵, or R⁶ include fluorine, chlorine, bromine, iodine, and the like.

In Formula (1), the optionally substituted alkyl represented by R⁴, R⁵, or R⁶ is not particularly limited. Examples include linear, branched, or cyclic (preferably linear or branched, and more preferably linear) alkyl groups containing 1 to 8, preferably 1 to 6, more preferably 1 to 4, even more preferably 1 to 3, and still more preferably 1 to 2 carbon atoms, and being optionally substituted with halogen (e.g., fluorine, chlorine, bromine, iodine), oxo, hydroxyl, alkoxy, optionally substituted amino, phenyl, and the like. The number of substituents is not particularly limited and is, for example, 0 to 6, preferably 0 to 3, and more preferably 0. Examples of the optionally substituted alkyl include methyl, ethyl, n-propyl, isopropyl, n-butyl, isobutyl, sec-butyl, t-butyl, perfluoromethyl, perfluoroethyl, and the like.

In Formula (1), the optionally substituted alkenyl represented by R⁴, R⁵, or R⁶ is not particularly limited. Examples include linear or branched (preferably linear) alkenyl groups containing 2 to 8, preferably 2 to 6, and more preferably 2 to 4 carbon atoms, and being optionally substituted with halogen (e.g., fluorine, chlorine, bromine, iodine), oxo, hydroxyl, alkoxy, optionally substituted amino, phenyl, and the like. The number of substituents is not particularly limited and is, for example, 0 to 6, preferably 0 to 3, and more preferably 0. Examples of the optionally substituted alkenyl include vinyl, allyl, 1-propenyl, isopropenyl, butenyl, pentenyl, hexenyl, and the like.

In Formula (1), the optionally substituted alkynyl represented by R⁴, R⁵, or R⁶ is not particularly limited. Examples include linear or branched (preferably linear) alkynyl groups containing 2 to 8, preferably 2 to 6, and more preferably 2 to 4 carbon atoms, and being optionally substituted with halogen (e.g., fluorine, chlorine, bromine, iodine), oxo, hydroxyl, alkoxy, optionally substituted amino, phenyl, and the like. The number of substituents is not particularly limited and is, for example, 0 to 6, preferably 0 to 3, and more preferably 0. Examples of the optionally substituted alkynyl include ethynyl, propynyl (e.g., 1-propynyl, 2-propynyl (propargyl)), butynyl, pentynyl, hexynyl, phenylacetylenyl, and the like.

In Formula (1), R⁴ is preferably hydrogen, optionally substituted alkyl, optionally substituted alkenyl, optionally substituted alkynyl, or the like, more preferably hydrogen, optionally substituted alkyl, optionally substituted alkenyl, or the like, and even more preferably optionally substituted alkyl, optionally substituted alkenyl, or the like.

In Formula (1), R⁵ is preferably hydrogen, optionally substituted alkyl, optionally substituted alkenyl, optionally substituted alkynyl, or the like, and more preferably hydrogen.

In Formula (1), R⁶ is preferably hydrogen, optionally substituted alkyl, optionally substituted alkenyl, optionally substituted alkynyl, or the like, and more preferably hydrogen.

In one embodiment, it is preferable that R⁴ be optionally substituted alkyl, optionally substituted alkenyl, or optionally substituted alkynyl, and R⁵ and R⁶ both be hydrogen atoms.

### 1-5. Preferable Formula

In one embodiment of the present invention, the compound represented by Formula (1) is preferably a compound represented by Formula (1A): 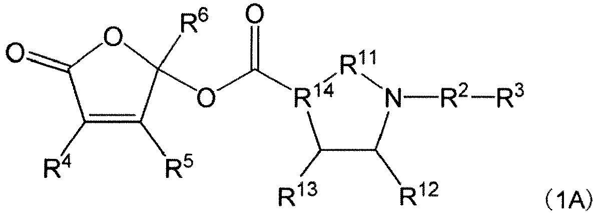
wherein R¹¹, R¹², R¹³, R¹⁴, R², R³, R⁴, R⁵, and R⁶ are as defined above, and more preferably a compound represented by Formula (1B) : 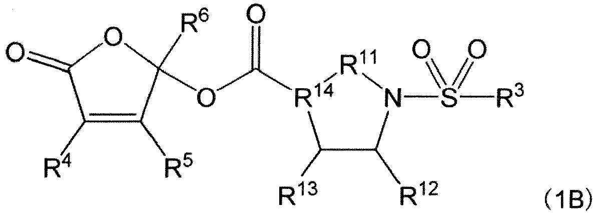
wherein R¹¹, R¹², R¹³, R¹⁴, R³, R⁴, R⁵, and R⁶ are as defined above, and even more preferably a compound represented by Formula (1C): 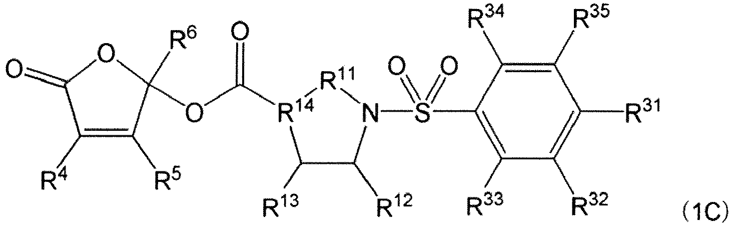
wherein R¹¹, R¹², R¹³, R¹⁴, R³¹, R³², R³³, R³⁴, R³⁵, R⁴, R⁵, and R⁶ are as defined above, and still more preferably a compound represented by Formula (1D): 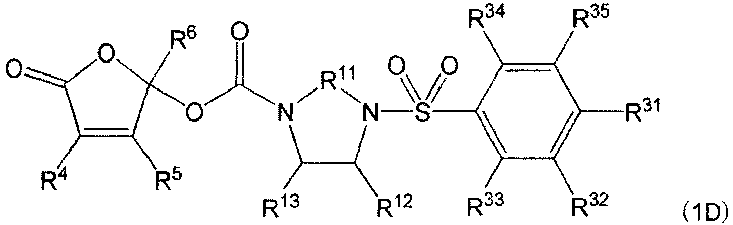
wherein R¹¹, R¹², R¹³, R³¹, R³², R³³, R³⁴, R³⁵, R⁴, R⁵, and R⁶ are as defined above.

### 1-6. Salt, hydrate, and solvate

The salts of the compounds represented by Formula (1) are not particularly limited as long as they are agriculturally acceptable salts. The salts may be either acid salts or basic salts. Examples of acid salts include inorganic acid salts such as hydrochloride, hydrobromide, sulfate, nitrate, and phosphate; and organic acid salts, such as acetate, propionate, tartrate, fumarate, maleate, malate, citrate, methanesulfonate, and p-toluenesulfonate. Examples of basic salts include alkali metal salts such as sodium salts and potassium salts; alkaline earth metal salts, such as calcium salts and magnesium salts; salts with ammonia; salts with organic amines, such as morpholine, piperidine, pyrrolidine, monoalkylamine, dialkylamine, trialkylamine, mono(hydroxyalkyl)amine, di(hydroxyalkyl)amine, and tri(hydroxyalkyl)amine; and the like.

The compound represented by Formula (1) can also be a hydrate or solvate. Examples of the solvent include agriculturally acceptable organic solvents (such as ethanol, glycerol, and acetic acid).

### 2. Production Method

The compound represented by Formula (1) can be synthesized by various methods.

### 2-1. Production Method 1

The compound represented by Formula (1) can be synthesized, for example, in accordance with the following reaction scheme or a scheme according to this scheme: 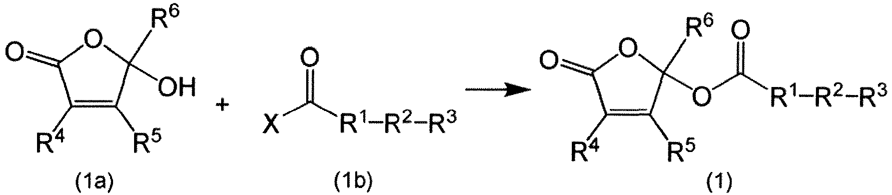 wherein R¹, R², R³, R⁴, R⁵, and R⁶ are as defined above, and X is a leaving group (for example, halogen, imidazolyl, alkoxy, or acyloxy, and preferably chlorine).

In this reaction, the compound represented by Formula (1) can be obtained by reacting the compound represented by Formula (1a) with the compound represented by Formula (1b).

From the viewpoint of the yield and the like, the amount of the compound represented by Formula (1a) is usually preferably 0.5 to 10 mol, more preferably 1.5 to 6 mol, and even more preferably 2 to 4 mol, per mole of the compound represented by Formula (1b).

This reaction is preferably performed in the presence of a catalyst. Examples of the catalyst include, but are not particularly limited to, N,N-dimethyl-4-aminopyridine (DMAP), triethylamine (Et₃N), and other base catalysts. These catalysts may be used alone or in a combination of two or more.

The amount of catalyst used varies depending on the type of catalyst. For example, the amount is about 0.1 to 10 mol per mole of the compound represented by Formula (1a).

This reaction is usually performed in the presence of a reaction solvent. Examples of the reaction solvent include, but are not particularly limited to, dichloromethane, acetonitrile, tetrahydrofuran, acetone, toluene, and the like, with dichloromethane and the like being preferable. These solvents may be used alone or in a combination of two or more.

In this reaction, in addition to the above components, additives can also be appropriately used as long as the progress of the reaction is not significantly impaired.

The reaction can be performed under heating, at room temperature, or under cooling, and is usually preferably performed at 0 to 50°C (in particular 0 to 30°C). The reaction time is not particularly limited and is usually 4 to 48 hours, and in particular 8 to 24 hours.

The progress of the reaction can be traced by chromatography or other usual methods. After completion of the reaction, the solvent is distilled off, and the product can be isolated and purified by chromatography, recrystallization, or other usual methods. The structure of the product can be identified by elemental analysis, MS (ESI-MS) analysis, IR analysis, ¹H-NMR, ¹³C-NMR, etc.

### 2-2. Production Method 2

The compound represented by Formula (1) can be synthesized, for example, in accordance with the following reaction scheme or a scheme according to this scheme: 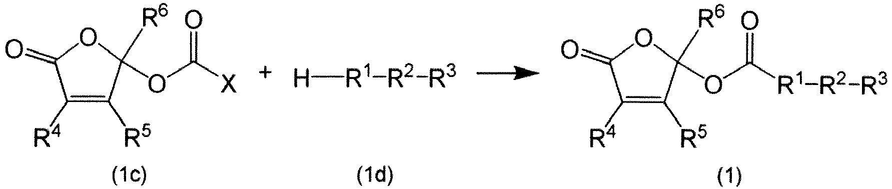 wherein R¹, R², R³, R⁴, R⁵, and R⁶ are as defined above, and X is a leaving group (for example, halogen, imidazolyl, alkoxy, acyloxy, or the like, preferably halogen, and more preferably chlorine).

In this reaction, the compound represented by Formula (1) can be obtained by reacting the compound represented by Formula (1c) with the compound represented by Formula (1d).

The reaction conditions of this reaction are the same as those stated above in the "2-1. Production Method 1" section.

### 3. Usage

The compound represented by Formula (1) exerts parasitic plant germination regulation action (in particular, parasitic plant germination-inducing action). Therefore, the compound represented by Formula (1), a salt thereof, a hydrate thereof, or a solvate thereof, can be used as an active ingredient of a parasitic plant germination regulator (in particular, a parasitic plant germination inducer).

If the parasitic plant germination regulator is applied, for example, to an environment where plants that serve as a host of parasitic plants are not present in surroundings, parasitic plants after germination cannot parasitize plants, and will wither and die, allowing the soil to be purified.

Examples of plants that serve as a host of parasitic plants include, but are not particularly limited to, corn, millet, sorghum, sugarcane, rice, beans, and the like.

The target parasitic plants are not particularly limited. Typical examples include plants of the genus *Striga.* Examples of plants of the genus *Striga* include *Striga hermonthica, Striga gesnerioides, Striga asiatica, Striga aequinoctialis, Striga angolensis, Striga angustifolia, Striga aspera, Striga bilabiata, Striga brachycalyx, Striga chrysantha, Striga dalzieli* , *Striga elegans, Striga forbesii, Striga gastonii, Striga gracillima, Striga hallaei, Striga hirsuta, Striga junodii, Striga klingii, Striga latericea, Striga lepidagathidis, Striga lutea, Striga macrantha, Striga passargei, Striga pinnatifida, Striga primuloides, Striga pubiflora, Striga yemenica,* and the like. Additionally, examples of parasitic plants targeted by the present invention include plants of the genus *Orobanche,* plants of the genus *Phelipanche,* and plants of the genus *Alectra,* and the like. Examples of plants of the genus *Orobanche* include *Orobanche cernua, Orobanche crenata, Orobanche cumana, Orobanche foetida, Orobanche minor,* and the like. Examples of plants of the genus *Phelipanche* include *Phelipanche aegyptiaca, Phelipanche ramosa,* and the like. Examples of plants of the genus Alectra include *Alectra orobanchoides, Alectra sessiliflora, Alectra vogelii,* and the like. The target parasitic plants are preferably plants of the genus *Striga,* plants of the genus *Orobanche,* and plants of the genus *Phelipanche,* more preferably plants of the genus *Striga* and plants of the genus *Orobanche,* and even more preferably *Striga hermonthica* and *Orobanche minor.*

The parasitic plant germination regulator of the present invention may consist of an active ingredient (the compound represented by Formula (1), a salt thereof, a hydrate thereof, or a solvate thereof). However, in addition to the above, the parasitic plant germination regulator of the present invention may further contain various additives, according to the dosage form, application mode, etc. The content of the active ingredient in the agent of the present invention is not particularly limited, and is specifically 0.0001 to 100 wt%, and preferably about 0.01 to 50 wt%.

The dosage form of the parasitic plant germination regulator of the present invention is not particularly limited as long as it is an agriculturally acceptable dosage form. Examples include liquids, solids, powders, granules, pellets, wettable powders, flowable agents, emulsions, pastes, dispersants, and the like.

The additives are not particularly limited as long as they are agriculturally acceptable additives. Examples include carriers, surfactants, thickeners, extenders, binders, vitamins, antioxidants, pH adjusters, volatilization inhibitors, dyes, and the like.

The application mode of the parasitic plant germination regulator of the present invention is not particularly limited as long as the active ingredient above can be brought into contact with parasitic plant seeds. Examples include an embodiment in which the agent of the present invention is sprayed, dripped, applied, mixed, etc. with respect to soil containing parasitic plant seeds.

### Examples

The present invention will be described in detail below based on Examples, but the present invention is not limited to these Examples.

### Example 1. Synthesis of Compound

Compounds represented by the following formula (6R) (compounds 1 to 9), compounds represented by the following formula (7R) (compounds 10 to 21), compounds represented by the following formula (8AB) (compounds 22 to 26), compounds represented by the following formula (9AB) (compounds 27 to 28), compounds represented by the following formula (10AB) (compounds 29 to 30), and compound 31 below were synthesized. 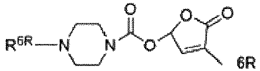 **R^{6R} =** 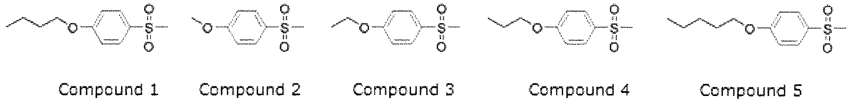 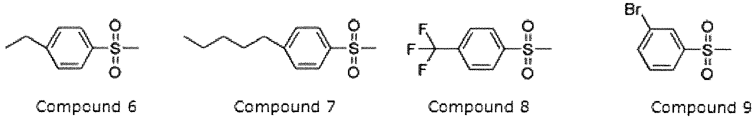 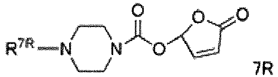 R^{7R} =

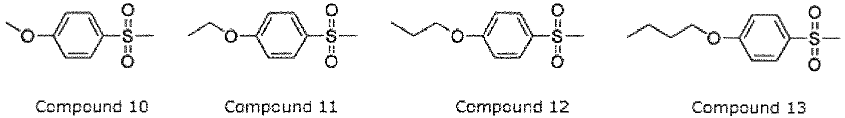

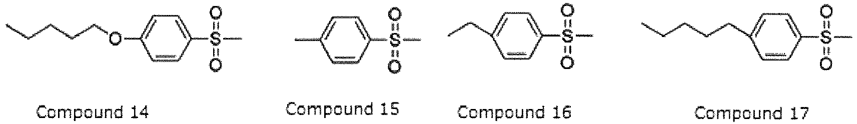

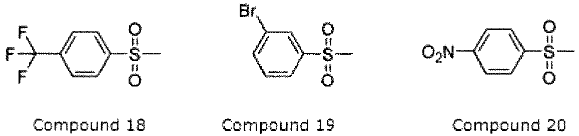

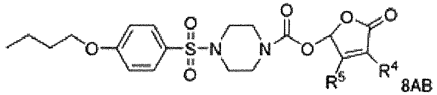

**R⁴=-CH₂CH₃, R**⁵=**-H** Compound 21

**R⁴=-H, R⁵=-CH₃** Compound 22

**R⁴=-CH₃, R⁵**=**-CH₃** Comoound 23

**R⁴**=**-CH₂CHCH₂(allyl)**, **R⁵=-H** Compound 24

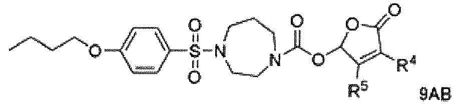

**R⁴=-CH₃, R⁵=-H** Compound 25

**R⁴=-H, R⁵=-H** Compound 26

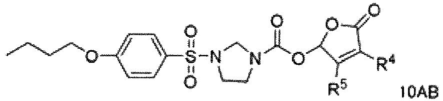

**R⁴=-CH₃, R⁵=-H** Compound 27

**R⁴=-H, R⁵=-H** Compound 28

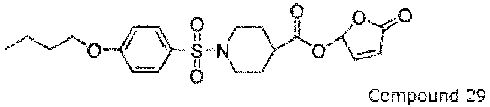

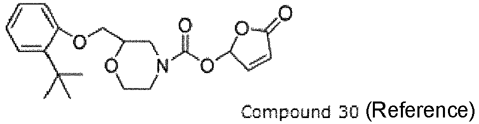

### Synthesis of Compound 1

Compound 1 was synthesized according to the following synthetic scheme. In the synthetic scheme, Et represents ethyl (the same applies hereinafter).

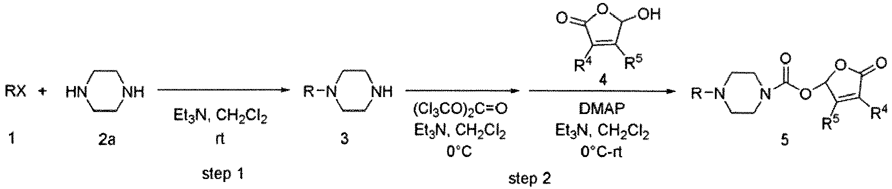

### Step: 1

4-Butoxybenzene-1-sulfonyl chloride (RX) (1a) (1.36 g, 5.5 mmol, 1 equiv.) and triethylamine (1.2 mL, 8.25 mmol, 1.5 equiv.) were added to a solution of piperazine (2a) (1.86 g, 22 mmol, 4 equiv.) in dichloromethane (55 mL), followed by stirring at room temperature overnight. Thereafter, the reaction liquid was quenched with saturated sodium hydrogen carbonate. The reaction mixture was extracted with dichloromethane (10 mL) (× 3), and dried over anhydrous Na₂SO₄. After the solvent was evaporated, the residue was purified by silica gel column chromatography (eluate: mixed solvent of chloroform/methanol), thus yielding white solid amine (3) (1.52 g, yield: 93%).

### Step: 2

A solution of triphosgene (0.15 g, 0.50 mmol, 1 equiv.) in dichloromethane (1.9 mL) was cooled to 0°C, and amine (3) (0.15 g, 0.50 mmol, 1 equiv.) and triethylamine (0.2 mL, 1.50 mmol, 3 equiv.) were added thereto, followed by stirring for 2 hours in an argon atmosphere. While the reaction liquid was maintained at 0°C, dichloromethane (5 mL), 5-hydroxy-3-methyl-2-furanone (4a) (0.17 g, 1.5 mmol, 3 equiv.), triethylamine (0.2 mL, 1.50 mmol, 3 equiv.), and N,N-dimethyl-4-aminopyridine (6.1 mg, 0.05 mmol, 10 mol%) were added thereto. The reaction liquid was stirred overnight while its temperature was slowly returned to room temperature. Thereafter, the reaction liquid was quenched with saturated sodium hydrogen carbonate, extracted with dichloromethane (5 mL) (× 3), and dried over anhydrous Na₂SO₄. After the solvent was evaporated, the residue was purified by silica gel column chromatography (eluate: a mixed solvent of hexane/ethyl acetate), thus yielding a white solid target product (5) (compound 1) (0.19 g, yield: 87%).
¹H NMR (600 MHz, CDCl₃) δ 7.65 (2H, dt, J = 9.0, 1.8 Hz), 6.99 (2H, dt, J = 9.0, 1.8 Hz), 6.85-6.83 (1H, m), 6.83-6.82 (1H, m), 4.03 (2H, t, J = 6.0 Hz), 3.76 (1H, d, J = 12.0 Hz), 3.65 (1H, d, J = 12.0 Hz), 3.47 (1H, t, J = 10.2 Hz), 3.43 (1H, t, J = 10.2 Hz), 3.19 (1H, br), 3.12 (1H, br), 2.84 (1H, t, J = 10.2 Hz), 2.79 (1H, t, J = 10.2 Hz), 1.97 (3H, s), 1.80 (2H, td, J = 7.2, 6.0 Hz), 1.51 (2H, sextet, J = 7.2 Hz), 0.99 (3H, t, J = 7.2 Hz); ¹³C NMR (151 MHz, CDCl₃) δ 171.1, 163.2, 152.4, 142.1, 134.7, 130.0, 126.4, 115.0, 93.9, 68.4, 45.8, 43.7, 43.4, 31.2, 19.3, 14.0, 10.8, one carbon atom was not found due to overlapping; IR (film) 2940, 2872, 1776, 1717, 1593, 1435, 1346, 1242, 1159, 1092, 1015, 955, 930 cm⁻¹; HRMS (ESI) Calcd for C₂₀H₂₆O₇N₂NaS⁺ ([M+Na]⁺) 461.1353, Found 461.1356.

### Synthesis of Compounds 2 to 26

According to the synthesis method for compound 1, synthesis was performed using appropriate material compounds.
Compound 2: White solid (145 mg, yield: 73%) . ¹H NMR (600 MHz, CDCl₃) δ 7.67 (2H, d, J = 8.4 Hz), 7.01 (2H, d, J = 8.4 Hz), 6.85-6.84 (1H, m), 6.83 (1H, brs), 3.89 (3H, s), 3.77 (1H, brd, J = 12.2 Hz), 3.66 (1H, brd, J = 12.2 Hz), 3.52-3.40 (2H, m), 3.19 (1H, brs), 3.13 (1H, brs), 2.84 (1H, t, J = 8.4 Hz), 2.79 (1H, t, J = 8.4 Hz), 1.97 (3H, s); ¹³C NMR (151 MHz, CDCl₃) δ 171.1, 163.4, 152.3, 142.1, 134.4, 129.8, 126.6, 114.5, 93.7, 55.7, 45.7, 43.5, 43.3, 10.6, one carbon atom was not found due to overlapping; IR (film) 2895, 2855, 1780, 1717, 1595, 1435, 1346, 1242, 1192, 1061, 1016, 955 cm⁻¹; HRMS (ESI) Calcd for C₁₇H₂₀O₇N₂NaS⁺ ([M+Na]⁺) 419.0883, Found 419.0871.
Compound 3: White solid (57 mg, yield: 82%). ¹H NMR (600 MHz, CDCl₃) δ 7.66 (2H, dt, J = 9.0, 1.8 Hz), 6.99 (2H, dt, J = 9.0, 1.8 Hz), 6.85 (1H, m), 6.82 (1H, m), 4.11 (2H, q, J = 6.9 Hz), 3.75 (1H, d, J = 11.4 Hz), 3.65 (1H, d, J = 11.4 Hz), 3.48 (1H, t, J = 9.0 Hz), 3.44 (1H, t, J = 9.0 Hz), 3.18 (1H, br), 3.12 (1H, br), 2.84 (1H, t, J = 9.0 Hz), 2.80 (1H, t, J = 9.0 Hz), 1.96 (3H, s), 1.45 (3H, t, J = 6.9 Hz); ¹³C NMR (151 MHz, CDCl₃) δ 171.2, 163.0, 152.4, 142.1, 134.6, 129.9, 126.4, 115.0, 93.8, 64.2, 45.8, 43.6, 43.4, 14.7, 10.7, one carbon atom was not found due to overlapping; IR (film) 2899, 1780, 1713, 1595, 1420, 1329, 1260, 1207, 1092, 1015, 955 cm⁻¹; HRMS (ESI) Calcd for C₁₈H₂₂O₇N₂NaS⁺ ([M+Na]⁺) 433.1040, Found 433.1039.
Compound 4: White solid (145 mg, yield: 68%). ¹H NMR (600 MHz, CDCl₃) δ 7.66 (2H, d, J = 9.0 Hz), 7.00 (2H, d, J = 9.0 Hz), 6.85 (1H, s), 6.83 (1H, s), 3.99 (2H, t, J = 7.2 Hz), 3.76 (1H, d, J = 12.6 Hz), 3.66 (1H, d, J = 12.6 Hz), 3.48 (1H, t, J = 9.6 Hz), 3.44 (1H, t, J = 9.6 Hz), 3.18 (1H, br), 3.12 (1H, br), 2.84 (1H, t, J = 9.6 Hz), 2.79 (1H, t, J = 9.6 Hz), 1.97 (3H, s), 1.85 (2H, sextet, J = 7.2 Hz), 1.06 (3H, t, J = 7.2 Hz); ¹³C NMR (151 MHz, CDCl₃) δ 171.2, 163.2, 152.4, 142.1, 134.7, 130.0, 126.4, 115.0, 93.9, 70.2, 45.8, 43.7, 43.4, 22.5, 10.8, 10.6, one carbon atom was not found due to overlapping; IR (film) 2941, 2899, 1775, 1707, 1591, 1439, 1344, 1242, 1159, 1092, 1011, 955, 934 cm⁻¹; HRMS (ESI) Calcd for C₁₉H₂₄O₇N₂NaS⁺ ([M+Na]⁺) 447.1196, Found 447.1195.
Compound 5: White solid (230 mg, yield: 990). ¹H NMR (600 MHz, CDCl₃) δ 7.65 (2H, d, J = 8.4 Hz), 6.99 (2H, d, J = 8.4 Hz), 6.85 (1H, brs), 6.82 (1H, brs), 4.02 (2H, t, J = 7.0 Hz), 3.76 (1H, d, J = 12.6 Hz), 3.66 (1H, d, J = 12.6 Hz), 3.48 (1H, t, J = 9.0 Hz), 3.44 (1H, t, J = 9.0 Hz), 3.18 (1H, br), 3.12 (1H, br), 2.84 (1H, t, J = 9.0 Hz), 2.79 (1H, t, J = 9.0 Hz), 1.96 (3H, s), 1.82 (2H, quintet, J = 7.0 Hz), 1.45 (2H, quintet, J = 7.0 Hz), 1.40 (2H, sextet, J = 7.0 Hz), 0.94 (3H, t, J = 7.0 Hz); ¹³C NMR (151 MHz, CDCl₃) δ 171.2, 163.2, 152.4, 142.1, 134.6, 129.9, 126.4, 115.0, 93.8, 68.7, 45.8, 43.6, 43.4, 28.8, 28.2, 22.5, 14.1, 10.7, one carbon atom was not found due to overlapping; IR (film) 2936, 2922, 1769, 1722, 1595, 1433, 1348, 1242, 1165, 1092, 1015, 957 cm⁻¹; HRMS (ESI) Calcd for C₂₁H₂₈O₇N₂NaS⁺ ([M+Na]⁺) 475.1509, Found 475.1507.
Compound 6: White solid (130 mg, yield: 66%). ¹H NMR (600 MHz, CDCl₃) δ 7.64 (2H, d, J = 8.4 Hz), 7.37 (2H, d, J = 8.4 Hz), 6.84 (1H, quintet, J = 1.2 Hz), 6.82 (1H, quintet, J = 1.2 Hz), 3.77 (1H, d, J = 12.3 Hz), 3.67 (1H, d, J = 12.3 Hz), 3.47 (1H, t, J = 11.4 Hz), 3.43 (1H, t, J = 11.4 Hz), 3.21 (1H, t, J = 6.6 Hz), 3.15 (1H, t, J = 6.6 Hz), 2.85 (1H, t, J = 8.8 Hz), 2.80 (1H, t, J = 8.8 Hz), 2.75 (2H, q, J = 7.8 Hz), 1.96 (3H, t, J = 1.2 Hz), 1.29 (3H, t, J = 7.8 Hz); ¹³C NMR (151 MHz, CDCl₃) δ 171.1, 152.4, 150.5, 142.1, 134.7, 132.5, 128.9, 128.0, 93.9, 45.8, 43.7, 43.5, 29.0, 15.2, 10.8, one carbon atom was not found due to overlapping; IR (film) 2967, 2926, 2855, 1778, 1719, 1435, 1350, 1242, 1165, 1125, 1092, 1015, 928 cm⁻¹; HRMS (ESI) Calcd for C₁₈H₂₂O₆N₂NaS⁺ ([M+Na]⁺) 417.1091, Found 417.1089.
Compound 7: White solid (220 mg, yield: 990). ¹H NMR (600 MHz, CDCl₃) δ 7.63 (2H, d, J = 8.4 Hz), 7.35 (2H, d, J = 8.4 Hz), 6.84 (1H, brs), 6.83 (1H, s), 3.77 (1H, d, J = 12.3 Hz), 3.67 (1H, d, J = 12.3 Hz), 3.48 (1H, t, J = 9.6 Hz), 3.44 (1H, t, J = 9.6 Hz), 3.21 (1H, t, J = 6.0 Hz), 3.15 (1H, t, J = 6.0 Hz), 2.86 (1H, t, J = 8.4 Hz), 2.81 (1H, t, J = 8.4 Hz), 2.68 (2H, t, J = 7.2 Hz), 1.96 (3H, s), 1.65 (2H, quintet, J = 7.2 Hz), 1.39-1.30 (4H, m), 0.91 (3H, t, J = 7.2 Hz); ¹³C NMR (151 MHz, CDCl₃) δ 171.1, 152.4, 149.3, 142.1, 134.7, 132.5, 129.4, 127.9, 93.9, 45.8, 43.7, 43.5, 36.0, 31.6, 30.8, 22.6, 10.8, one carbon atom was not found due to overlapping; IR (film) 2928, 2857, 1780, 1721, 1435, 1350, 1242, 1167, 1126, 1092, 1015, 955 cm⁻¹; HRMS (ESI) Calcd for C₂₁H₂₈O₆N₂NaS⁺ ([M+Na]⁺) 459.1560, Found 459.1558.
Compound 8: White solid (220 mg, yield: 99%). ¹H NMR (600 MHz, CDCl₃) δ 7.88 (2H, d, J = 8.2 Hz), 7.84 (2H, d, J = 8.2 Hz), 6.84 (1H, brs), 6.83 (1H, brs), 3.77 (1H, d, J = 12.0 Hz), 3.68 (1H, d, J = 12.0 Hz), 3.52 (1H, t, J = 10.2 Hz), 3.48 (1H, t, J = 10.2 Hz), 3.23 (1H, br), 3.17 (1H, br), 2.95 (1H, t, J = 10.2 Hz), 2.90 (1H, t, J = 10.2 Hz), 1.97 (3H, s); ¹³C NMR (151 MHz, CDCl₃) δ 171.1, 152.4, 142.0, 139.3, 135.2 (q, J_{C-F} = 33.2 Hz), 134.8, 128.3, 126.7 (q, J_{C-F} = 4.2 Hz), 123.2 (q, J_{C-F} = 273.2 Hz), 93.9, 45.7, 43.7, 43.4, 10.8, one carbon atom was not found due to overlapping; IR (film) 2928, 2859, 1780, 1721, 1437, 1323, 1244, 1171, 1128, 1063, 1015, 955 cm⁻¹; HRMS (ESI) Calcd for C₁₇H₁₇O₆N₂F₃NaS⁺ ([M+Na]⁺) 457.0652, Found 457.0646.
Compound 9: White solid (37 mg, yield: 83%) . ¹H NMR (600 MHz, CDCl₃) δ 7.89-7.87 (1H, m), 7.77 (1H, d, J = 8.2 Hz), 7.67 (1H, d, J = 8.2 Hz), 7.44 (1H, t, J = 8.2 Hz), 6.86-6.84 (1H, m), 6.82 (1H, brs), 3.75 (1H, d, J = 12.6 Hz), 3.65 (1H, d, J = 12.6 Hz), 3.52 (1H, t, J = 11.4 Hz), 3.47 (1H, t, J = 11.4 Hz), 3.20 (1H, brs), 3.13 (1H, brs), 2.94 (1H, t, J = 7.0 Hz), 2.89 (1H, t, J = 7.0 Hz), 1.97 (3H, s); ¹³C NMR (151 MHz, CDCl₃) δ 171.1, 152.4, 142.0, 137.4, 136.5, 134.7, 131.0, 130.6, 126.3, 123.6, 93.9, 45.8, 43.7, 43.4, 10.8, one carbon atom was not found due to overlapping; IR (film) 2924, 2862, 1776, 1717, 1435, 1352, 1240, 1169, 1125, 1096, 1015, 953 cm⁻¹; HRMS (ESI) Calcd for C₁₆H₁₇O₆N₂⁷⁹BrNaS⁺ ([M+Na]⁺) 466.9883, Found 466.9876.
Compound 10: White solid (21 mg, yield: 55%) . ¹H NMR (600 MHz, CDCl₃) δ 7.68 (2H, dt, J = 9.0, 2.4 Hz), 7.27 (1H, dd, J = 7.2, 1.8 Hz), 7.02 (2H, dt, J = 9.0, 2.4 Hz), 6.96 (1H, t, J = 1.8 Hz), 6.29 (1H, d, J = 7.2 Hz), 3.89 (3H, s), 3.77 (1H, d, J = 12.6 Hz), 3.67 (1H, d, J = 12.6 Hz), 3.49 (1H, t, J = 11.0 Hz), 3.45 (1H, t, J = 11.0 Hz), 3.20 (1H, br), 3.14 (1H, br), 2.85 (1H, t, J = 11.0 Hz), 2.81 (1H, t, J = 11.0 Hz); ¹³C NMR (151 MHz, CDCl₃) δ 169.7, 163.6, 152.2, 149.7, 130.0, 126.8, 125.5, 114.7, 95.4, 55.8, 45.8, 43.7, 43.5, one carbon atom was not found due to overlapping; IR (film) 2982, 2847, 1792, 1719, 1595, 1499, 1437, 1348, 1242, 1161, 1092, 1018, 926 cm⁻¹; HRMS (ESI) Calcd for C₁₆H₁₈O₇N₂NaS⁺ ([M+Na]⁺) 405.0727, Found 405.0721.
Compound 11: White solid (38 mg, yield: 56%). ¹H NMR (600 MHz, CDCl₃) δ 7.66 (2H, dt, J = 8.7, 2.4 Hz), 7.27 (1H, dd, J = 5.4, 1.2 Hz), 6.99 (2H, dt, J = 8.7, 2.4 Hz), 6.96 (1H, t, J = 1.2 Hz), 6.29 (1H, dd, J = 5.4, 1.2 Hz), 4.11 (2H, q, J = 7.5 Hz), 3.77 (1H, d, J = 12.6 Hz), 3.67 (1H, d, J = 12.6 Hz), 3.49 (1H, t, J = 10.5 Hz), 3.45 (1H, t, J = 10.5 Hz), 3.19 (1H, brs), 3.13 (1H, brs), 2.85 (1H, t, J = 10.5 Hz), 2.80 (1H, t, J = 10.5 Hz), 1.57 (3H, s), 1.46 (3H, t, J = 7.5 Hz); ¹³C NMR (151 MHz, CDCl₃) δ 169.7, 163.0, 152.2, 149.8, 130.0, 126.5, 125.5, 115.0, 95.4, 64.2, 45.8, 43.7, 43.5, 14.8, one carbon atom was not found due to overlapping; IR (film) 2984, 1792, 1717, 1595, 1435, 1348, 1242, 1161, 1090, 1018, 924 cm⁻¹; HRMS (ESI) Calcd for C₁₇H₂₀O₇N₂NaS⁺ ([M+Na]⁺) 419.0883, Found 419.0877.
Compound 12: White solid (137 mg, yield: 67%). ¹H NMR (600 MHz, CDCl₃) δ 7.66 (2H, d, J = 8.7 Hz), 7.28 (1H, dd, J = 6.0, 1.2 Hz), 7.00 (2H, d, J = 8.7 Hz), 6.96 (1H, t, J = 1.2 Hz), 6.29 (1H, dd, J = 6.0, 1.2 Hz), 3.99 (2H, t, J = 7.2 Hz), 3.76 (1H, d, J = 12.0 Hz), 3.66 (1H, d, J = 12.0 Hz), 3.49 (1H, t, J = 10.5 Hz), 3.45 (1H, t, J = 10.5 Hz), 3.19 (1H, br), 3.13 (1H, br), 2.85 (1H, t, J = 10.5 Hz), 2.81 (1H, t, J = 10.5 Hz), 1.85 (2H, sextet, J = 7.2 Hz), 1.06 (3H, t, J = 7.2 Hz); ¹³C NMR (151 MHz, CDCl₃) δ 169.7, 163.2, 152.1, 149.8, 130.0, 126.4, 125.5, 115.0, 95.3, 70.1, 45.8, 43.7, 43.5, 22.5, 10.6, one carbon atom was not found due to overlapping; IR (film) 2936, 2903, 1790, 1717, 1593, 1435, 1392, 1329, 1240, 1219, 1088, 1059, 1016, 972, 925 cm⁻¹; HRMS (ESI) Calcd for C₁₈H₂₂O₇N₂NaS⁺ ([M+Na]⁺) 433.1040, Found 433.1042.
Compound 13: White solid (94 mg, yield: 44%) . ¹H NMR (600 MHz, CDCl₃) δ 7.65 (2H, d, J = 9.0 Hz), 7.27 (1H, d, J = 6.0 Hz), 6.99 (2H, d, J = 9.0 Hz), 6.96 (1H, m), 6.28 (1H, d, J = 6.0 Hz), 4.03 (2H, t, J = 7.2 Hz), 3.76 (1H, d, J = 10.2 Hz), 3.66 (1H, d, J = 10.8 Hz), 3.49 (1H, t, J = 9.0 Hz), 3.44 (1H, t, J = 9.0 Hz), 3.19 (1H, br), 3.12 (1H, br), 2.85 (1H, t, J = 9.0 Hz), 2.81 (1H, t, J = 9.0 Hz), 1.80 (2H, quintet, J = 7.2 Hz), 1.51 (2H, sextet, J = 7.2 Hz), 0.99 (3H, t, J = 7.2 Hz); ¹³C NMR (151 MHz, CDCl₃) δ 169.6, 163.2, 152.2, 149.7, 130.0, 126.4, 125.5, 115.0, 95.4, 68.4, 45.8, 43.7, 43.5, 31.2, 19.3, 13.9, one carbon atom was not found due to overlapping; IR (film) 2961, 2934, 2872, 1792, 1719, 1595, 1435, 1348, 1242, 1161, 1092 cm⁻¹; HRMS (ESI) Calcd for C₁₉H₂₄O₇N₂NaS⁺ ([M+Na]⁺) 447.1196, Found 447.1193 .
Compound 14: White solid (150 mg, yield: 68%). ¹H NMR (600 MHz, CDCl₃) δ 7.66 (2H, d, J = 7.2 Hz), 7.27 (1H, dd, J = 5.4, 1.2 Hz), 6.99 (2H, d, J = 9.0 Hz), 6.96 (1H, brs), 6.29 (1H, d, J = 5.4 Hz), 4.02 (2H, t, J = 7.2 Hz), 3.77 (1H, brd, J = 12.6 Hz), 3.66 (1H, brd, J = 12.6 Hz), 3.49 (1H, t, J = 12.6 Hz), 3.45 (1H, t, J = 12.0 Hz), 3.19 (1H, t, J = 5.4 Hz), 3.13 (1H, t, J = 5.4 Hz), 2.85 (1H, t, J = 8.4 Hz), 2.80 (1H, t, J = 8.4 Hz), 1.82 (2H, quintet, J = 7.2 Hz), 1.45 (2H, quintet, J = 7.2 Hz), 1.40 (2H, sextet, J = 7.2 Hz), 0.94 (3H, t, J = 7.2 Hz); ¹³C NMR (151 MHz, CDCl₃) δ 169.7, 163.2, 152.2, 149.7, 130.0, 126.4, 125.5, 115.0, 95.4, 68.7, 45.8, 43.7, 43.5, 28.9, 28.2, 22.5, 14.1, one carbon atom was not found due to overlapping; IR (film) 2934, 2870, 1790, 1753, 1595, 1433, 1350, 1281, 1217, 1184, 1140, 1092, 1016, 966 cm⁻¹; HRMS (ESI) Calcd for C₂₀H₂₆O₇N₂NaS⁺ ([M+Na]⁺) 461.1353, Found 461.1354.
Compound 15: White solid (24 mg, yield: 66%). ¹H NMR (600 MHz, CDCl₃) δ 7.62 (2H, d, J = 8.4 Hz), 7.35 (2H, d, J = 8.4 Hz), 7.27 (1H, d, J = 5.4 Hz), 6.95 (1H, s), 6.28 (1H, d, J = 5.4 Hz), 3.76 (1H, d, J = 10.2 Hz), 3.66 (1H, d, J = 10.2 Hz), 3.48 (1H, t, J = 10.2 Hz), 3.45 (1H, t, J = 10.2 Hz), 3.20 (1H, brs), 3.14 (1H, brs), 2.85 (1H, t, J = 8.4 Hz), 2.81 (1H, t, J = 8.4 Hz), 2.45 (3H, s); ¹³C NMR (151 MHz, CDCl₃) δ 169.6, 152.1, 149.8, 144.4, 132.2, 130.1, 127.8, 125.4, 95.3, 45.8, 43.7, 43.5, 21.7, one carbon atom was not found due to overlapping; IR (film) 2924, 2855, 1792, 1721, 1435, 1348, 1242, 1165, 1125, 1088, 1018, 928 cm⁻¹; HRMS (ESI) Calcd for C₁₆H₁₈O₆N₂NaS⁺ ([M+Na]⁺) 389.0778, Found 389.0785.
Compound 16: White solid (110 mg, yield: 58%) . ¹H NMR (600 MHz, CDCl₃) δ 7.65 (2H, d, J = 8.4 Hz), 7.37 (2H, d, J = 8.4 Hz), 7.27 (2H, d, J = 6.0 Hz), 6.96 (1H, s), 6.29 (1H, d, J = 6.0 Hz), 3.78 (1H, d, J = 12.0 Hz), 3.67 (1H, d, J = 12.0 Hz), 3.48 (1H, t, J = 9.3 Hz), 3.44 (1H, t, J = 9.3 Hz), 3.22 (1H, t, J = 6.6 Hz), 3.16 (1H, t, J = 6.6 Hz), 2.86 (1H, t, J = 8.4 Hz), 2.82 (1H, t, J = 8.4 Hz), 2.75 (2H, q, J = 7.8 Hz), 1.29 (3H, t, J = 7.8 Hz); ¹³C NMR (151 MHz, CDCl₃) δ 169.6, 152.2, 150.5, 149.7, 132.5, 128.9, 128.0, 125.5, 95.4, 45.8, 43.8, 43.5, 29.0, 15.2, one carbon atom was not found due to overlapping; IR (film) 2974, 2866, 1792, 1721, 1435, 1348, 1242, 1165, 1090, 1018, 928 cm⁻¹; HRMS (ESI) Calcd for C₁₇H₂₀O₆N₂NaS⁺ ([M+Na]⁺) 403.0934, Found 403.0933.
Compound 17: White solid (138 mg, yield: 65%) . ¹H NMR (600 MHz, CDCl₃) δ 7.64 (2H, d, J = 7.5 Hz), 7.35 (2H, d, J = 7.5 Hz), 7.27 (1H, d, J = 6.0 Hz), 6.96 (1H, s), 6.29 (1H, d, J = 6.0 Hz), 3.77 (1H, d, J = 12.6 Hz), 3.67 (1H, d, J = 12.6 Hz), 3.49 (1H, t, J = 10.2 Hz), 3.45 (1H, t, J = 10.2 Hz), 3.21 (1H, t, J = 7.2 Hz), 3.16 (1H, t, J = 7.2 Hz), 2.87 (1H, t, J = 8.4 Hz), 2.82 (1H, t, J = 8.4 Hz), 2.68 (2H, t, J = 7.8 Hz), 1.65 (2H, quintet, J = 7.8 Hz), 1.39-1.30 (4H, m), 0.91 (3H, t, J = 7.8 Hz); ¹³C NMR (151 MHz, CDCl₃) δ 169.6, 152.2, 149.7, 149.3, 132.5, 129.4, 127.9, 125.5, 95.4, 45.8, 43.8, 43.6, 36.0, 31.6, 30.8, 22.6, 14.1, one carbon atom was not found due to overlapping; IR (film) 2926, 2860, 1794, 1721, 1435, 1350, 1242, 1165, 1125, 1090, 1016, 926 cm⁻¹; HRMS (ESI) Calcd for C₂₀H₂₆O₆N₂NaS⁺ ([M+Na]⁺) 445.1404, Found 445.1408.
Compound 18: White solid (109 mg, yield: 52%). ¹H NMR (600 MHz, CDCl₃) δ 7.89 (2H, d, J = 8.7 Hz), 7.84 (2H, d, J = 8.7 Hz), 7.27 (1H, dd, J = 5.4, 1.2 Hz), 6.96 (1H, s), 6.29 (1H, d, J = 5.4 Hz), 3.78 (1H, d, J = 12.3 Hz), 3.68 (1H, d, J = 12.3 Hz), 3.53 (1H, t, J = 7.8 Hz), 3.49 (1H, t, J = 7.8 Hz), 3.24 (1H, t, J = 4.0 Hz), 3.18 (1H, t, J = 4.0 Hz), 2.96 (1H, t, J = 8.2 Hz), 2.92 (1H, t, J = 8.2 Hz) ; ¹³C NMR (151 MHz, CDCl₃) δ 169.6, 152.1, 149.6, 139.3, 135.2 (q, J_{C-F}= 33.2 Hz), 128.3, 126.7 (q, J_{C-F} = 4.4 Hz), 125.6, 123.2 (q, J_{C-F}= 273.3 Hz), 95.4, 45.7, 43.7, 43.5, one carbon atom was not found due to overlapping; IR (film) 2934, 2920, 2874, 1792, 1722, 1595, 1435, 1323, 1242, 1165, 1126, 1090, 1063, 957 cm⁻¹; HRMS (ESI) Calcd for C₁₆H₁₅O₆N₂F₃NaS⁺ ([M+Na]⁺) 443.0495, Found 443.0494.
Compound 19: White solid (38 mg, yield: 88%). ¹H NMR (600 MHz, CDCl₃) δ 7.89-7.86 (1H, m), 7.78-7.75 (1H, m), 7.67 (1H, d, J = 7.2 Hz), 7.45 (1H, t, J = 7.8 Hz), 7.28 (1H, d, J = 6.0 Hz), 6.96 (1H, brs), 6.30-6.29 (1H, m), 3.76 (1H, brs), 3.66 (1H, brs), 3.53 (1H, t, J = 11.0 Hz), 3.48 (1H, t, J = 11.0 Hz), 3.21 (1H, brs), 3.15 (1H, brs), 2.95 (1H, t, J = 8.4 Hz), 2.90 (1H, t, J = 8.4 Hz); ¹³C NMR (151 MHz, CDCl₃) δ 169.6, 152.2, 149.7, 137.4, 136.5, 131.0, 130.6, 126.3, 125.5, 123.6, 95.4, 45.8, 43.7, 43.5, one carbon atom was not found due to overlapping; IR (film) 2922, 2862, 1792, 1721, 1435, 1352, 1242, 1171, 1126, 1086, 1018, 931 cm⁻¹; HRMS (ESI) Calcd for C₁₅H₁₅O₆N₂⁷⁹BrNaS⁺ ([M+Na]⁺) 452.9726, Found 452.9723.
Compound 20: White solid (160 mg, yield: 42%) . ¹H NMR (600 MHz, CDCl₃) δ 8.42 (2H, d, J = 8.7 Hz), 7.94 (2H, d, J = 8.7 Hz), 7.27 (1H, d, J = 5.4 Hz), 6.95 (1H, s), 6.29 (1H, d, J = 5.4 Hz), 3.81 (1H, d, J = 12.6 Hz), 3.70 (1H, d, J = 12.6 Hz), 3.52 (1H, t, J = 11.4 Hz), 3.48 (1H, t, J = 11.4 Hz), 3.28 (1H, br), 3.22 (1H, br), 2.96 (1H, t, J = 7.8 Hz), 2.92 (1H, t, J = 7.8 Hz); ¹³C NMR (151 MHz, CDCl₃) δ 169.5, 152.1, 150.7, 149.6, 141.6, 129.0, 125.6, 124.8, 95.4, 45.7, 43.8, 43.5, one carbon atom was not found due to overlapping; IR (film) 2981, 2855, 1794, 1719, 1531, 1437, 1352, 1244, 1171, 1088, 1018, 932 cm⁻¹; HRMS (ESI) Calcd for C₁₅H₁₅O₈N₃NaS⁺ ([M+Na]⁺) 420.0472, Found 420.0466.
Compound 21: White solid (7 mg, yield: 460). ¹H NMR (600 MHz, CDCl₃) δ 7.65 (2H, d, J = 9.0 Hz), 6.99 (2H, d, J = 9.0 Hz), 6.85-6.83 (1H, m), 6.81-6.80 (1H, m), 4.03 (2H, t, J = 6.6 Hz), 3.76 (1H, d, J = 12.6 Hz), 3.67 (1H, d, J = 12.6 Hz), 3.48 (1H, t, J = 9.6 Hz), 3.44 (1H, t, J = 9.6 Hz), 3.19 (1H, t, J = 6.6 Hz), 3.13 (1H, t, J = 6.6 Hz), 2.84 (1H, t, J = 9.6 Hz), 2.79 (1H, t, J = 9.6 Hz), 2.34 (2H, q, J = 7.4 Hz), 1.80 (2H, quintet, J = 6.6 Hz), 1.51 (2H, sextet, J = 6.6 Hz), 1.18 (3H, t, J = 7.4 Hz), 0.99 (3H, t, J = 6.6 Hz); ¹³C NMR (151 MHz, CDCl₃) δ 170.7, 163.2, 152.4, 140.7, 140.6, 130.0, 126.4, 115.0, 94.1, 68.4, 45.8, 43.7, 43.4, 31.2, 19.3, 18.9, 13.9, 11.5, one carbon atom was not found due to overlapping; IR (film) 2961, 2936, 2874, 1778, 1721, 1595, 1464, 1435, 1350, 1242, 1161, 1125, 1092, 1020, 947 cm⁻¹; HRMS (ESI) Calcd for C₂₁H₂₈O₇N₂NaS⁺ ([M+Na]⁺) 475.1509, Found 475.1505.
Compound 22: White solid (58 mg, yield: 66%) . ¹H NMR (600 MHz, CDCl₃) δ 7.65 (2H, d, J = 9.0 Hz), 7.00 (2H, d, J = 9.0 Hz), 6.74 (1H, brs), 5.93 (1H, brs), 4.03 (2H, t, J = 7.2 Hz), 3.81 (1H, d, J = 10.4 Hz), 3.70 (1H, d, J = 10.4 Hz), 3.50-3.39 (2H, m), 3.25 (1H, brs), 3.18 (1H, brs), 2.80 (1H, t, J = 8.7 Hz), 2.76 (1H, t, J = 8.7 Hz), 2.05 (3H, s), 1.80 (2H, quintet, J = 7.2 Hz), 1.51 (2H, sextet, d, J = 7.2 Hz), 0.99 (3H, t, J = 7.8 Hz); ¹³C NMR (151 MHz, CDCl₃) δ 169.9, 163.2, 162.7, 152.5, 129.9, 126.3, 119.7, 115.0, 96.1, 68.3, 45.8, 43.6, 43.5, 31.1, 19.3, 13.9, 13.4, one carbon atom was not found due to overlapping; IR (film) 2961, 2932, 2872, 1792, 1717, 1593, 1435, 1348, 1240, 1159, 1088, 1022, 978 cm⁻¹; HRMS (ESI) Calcd for C₂₀H₂₆O₇N₂NaS⁺ ([M+Na]⁺) 461.1353, Found 461.1349.
Compound 23: White solid (66 mg, yield: 73%) . ¹H NMR (600 MHz, CDCl₃) δ 7.65 (2H, d, J = 9.0 Hz), 6.99 (2H, d, J = 9.0 Hz), 6.67 (1H, s), 4.03 (2H, t, J = 6.6 Hz), 3.82 (1H, d, J = 12.3 Hz), 3.70 (1H, d, J = 12.3 Hz), 3.44 (1H, t, J = 10.4 Hz), 3.40 (1H, t, J = 10.4 Hz), 3.25 (1H, d, J = 9.3 Hz), 3.18 (1H, d, J = 9.3 Hz), 2.78 (1H, t, J = 9.0 Hz), 2.73 (1H, t, J = 9.0 Hz), 1.93 (3H, s), 1.85 (3H, s), 1.80 (2H, quintet, J = 6.6 Hz), 1.51 (2H, sextet, J = 6.6 Hz), 0.99 (3H, t, J = 6.6 Hz); ¹³C NMR (151 MHz, CDCl₃) δ 171.7, 163.2, 153.3, 152.8, 129.9, 127.4, 126.4, 115.0, 95.4, 68.4, 45.8, 43.7, 43.5, 31.2, 19.3, 13.9, 11.5, 8.7, one carbon atom was not found due to overlapping; IR (film) 2959, 2928, 2872, 1775, 1722, 1595, 1435, 1348, 1310, 1240, 1161, 1123, 1084, 989 cm⁻¹; HRMS (ESI) Calcd for C₂₁H₂₈O₇N₂NaS⁺ ([M+Na]⁺) 475.1509, Found 475.1506.
Compound 24: White solid (155 mg, yield: 67%). ¹H NMR (600 MHz, CDCl₃) δ 7.65 (2H, d, J = 9.0 Hz), 6.99 (2H, d, J = 9.0 Hz), 6.86-6.83 (2H, m), 5.85 (1H, ddt, J = 16.8, 10.8, 6.9 Hz), 5.20 (1H, dd, J = 16.8, 1.2 Hz), 5.19 (1H, dd, J = 10.8, 1.2 Hz), 4.03 (2H, t, J = 7.2 Hz), 3.75 (1H, d, J = 12.6 Hz), 3.66 (1H, d, J = 12.6 Hz), 3.48 (1H, t, J = 9.6 Hz), 3.44 (1H, t, J = 9.6 Hz), 3.17 (1H, br), 3.11 (1H, br), 3.06 (2H, d, J = 6.9 Hz), 2.85 (1H, t, J = 7.8 Hz), 2.80 (1H, t, J = 7.8 Hz), 1.80 (2H, quintet, J = 7.2 Hz), 1.51 (2H, sextet, J = 7.2 Hz), 0.99 (3H, t, J = 7.2 Hz); ¹³C NMR (151 MHz, CDCl₃) δ 170.3, 163.2, 152.3, 142.1, 137.5, 132.1, 129.9, 126.4, 118.9, 115.0, 94.0, 68.4, 45.8, 43.7, 43.4, 31.2, 29.6, 19.3, 13.9, one carbon atom was not found due to overlapping; IR (film) 2961, 2932, 2870, 1778, 1722, 1595, 1497, 1437, 1348, 1242, 1159, 1092, 1015, 964 cm⁻¹; HRMS (ESI) Calcd for C₂₂H₂₈O₇N₂NaS⁺ ([M+Na]⁺) 487.1509, Found 487.1493.
Compound 25: White solid (113 mg, yield: 83%). ¹H NMR (600 MHz, CDCl₃) mixture of rotamers δ 7.71-7.66 (2+2H, m), 6.97 (2+2H, d, J = 8.4 Hz), 6.89 (1H, q, J = 1.2 Hz), 6.88 (1H, q, J = 1.2 Hz), 6.87 (1H, s), 6.86 (1H, s), 4.02 (2+2H, t, J = 6.8 Hz), 3.74-3.67 (1H, m), 3.65-3.39 (5+6H, m), 3.18 (1H, ddd, J = 13.8, 7.8, 3.6 Hz), 3.12-3.01 (1+2H, m), 2.01-1.93 (4+4H, m), 1.93-1.86 (1+1H, m), 1.79 (2+2H, quintet, J = 6.6 Hz), 1.50 (2+2H, sextet, J = 6.8 Hz), 0.98 (3+3H, t, J = 6.8 Hz); ¹³C NMR (151 MHz, CDCl₃) mixture of rotamers δ 171.3₂, 171.2₉, 162.7, 153.6, 153.2, 142.4, 142.3, 134.5, 134.4, 130.4₀, 130.3₇, 129.0₇, 129.0₅, 114.9, 93.8, 68.3, 49.8, 49.7, 48.9, 47.8, 47.7, 46.5, 46.0, 31.2, 28.3, 27.8, 19.3, 13.9, 10.7; IR (film) 2957, 2872, 1780, 1721, 1713, 1595, 1423, 1331, 1256, 1153, 1092, 1013, 959 cm⁻¹; HRMS (ESI) Calcd for C₂₁H₂₈O₇N₂NaS⁺ ([M+Na]⁺) 475.1509, Found 475.1510.
Compound 26: Colorless oil (39 mg, yield: 29%). ¹H NMR (600 MHz, CDCl₃) mixture of rotamers δ 7.69 (2H, dd, J = 9.0, 2.4 Hz), 7.68 (2H, dd, J = 9.0, 2.4 Hz), 7.33 (1H, d, J = 6.0 Hz), 7.31 (1H, d, J = 6.0 Hz), 7.00 (1H, s), 6.99 (1H, s), 6.97 (2+2H, d, J = 9.0 Hz), 6.29 (1+1H, d, J = 6.0 Hz), 4.02 (2+2H, t, J = 6.6 Hz), 3.75-3.69 (1H, m), 3.64 (1H, dt, J = 13.8, 6.6 Hz), 3.60-3.41 (5+5H, m), 3.18 (1H, ddd, J = 13.8, 7.8, 3.0 Hz), 3.13-3.00 (1+2H, m), 2.04-1.85 (2+2H, m), 1.79 (2+2H, quintet, J = 7.2 Hz), 1.50 (2+2H, sextet, J = 7.2 Hz), 0.98 (3+3H, t, J = 7.2 Hz); ¹³C NMR (151 MHz, CDCl₃) mixture of rotamers δ 169.9, 169.8, 162.7, 153.4, 153.0, 150.1, 150.0, 130.4₀, 130.3₇, 129.0₉, 129.0₆, 125.4, 125.3, 115.0, 95.4, 68.3, 49.7₁, 49.6₇, 48.9, 47.9, 47.7, 46.7, 46.1, 31.2, 28.3, 27.9, 19.3, 13.9; IR (film) 2959, 2872, 1790, 1715, 1593, 1472, 1423, 1331, 1254, 1152, 1088, 1015 cm⁻¹; HRMS (ESI) Calcd for C₂₀H₂₆O₇N₂NaS⁺ ([M+Na]⁺) 461.1353, Found 461.1352.

### Synthesis of Compound 27

Compound 27 was synthesized according to the following synthetic scheme.

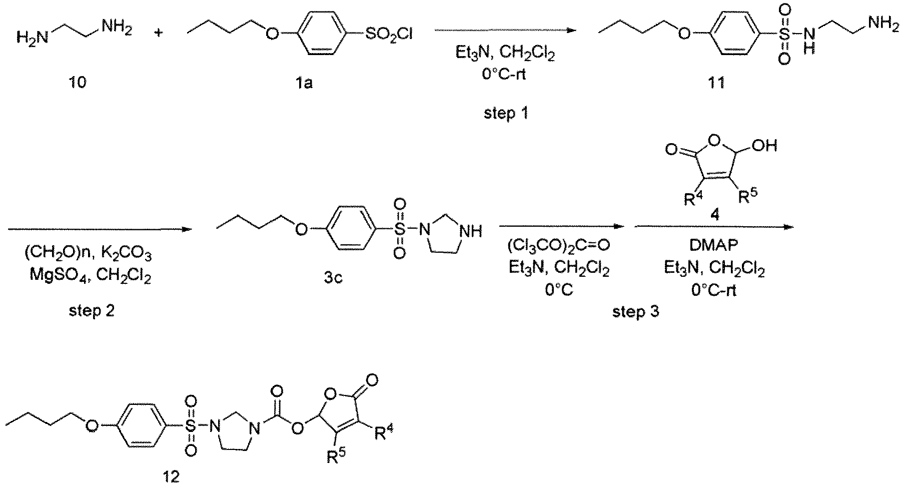

### Step: 1

A solution of ethylenediamine (10) (4.0 mL, 60 mmol, 5 equiv.) in dichloromethane (120 mL) was cooled to 0°C, and 4-butoxybenzene-1-sulfonyl chloride (1a) (3.0 g, 12 mmol, 1 equiv.) and triethylamine (2.0 mL, 14.4 mmol, 1.2 equiv.) were added thereto. The reaction liquid was stirred for 1 hour while its temperature was slowly returned to room temperature. The solvent was evaporated, diluted in diethyl ether (10 mL), and washed with dilute hydrochloric acid (1N, 5 mL) (× 3). The aqueous layer was adjusted to a pH of 10 with an aqueous potassium carbonate solution, then extracted with ethyl acetate (5 mL) (× 3), dried over anhydrous Na₂SO₄, and concentrated, thus yielding white solid amine (11) (1.5 g, yield: 47%).

### Step: 2

Amine (11), (27 mg, 0.10 mmol, 1 equiv.), paraformaldehyde (15 mg, 0.50 mmol, 5 equiv.), potassium carbonate (48 mg, 0.35 mmol, 3.5 equiv.), and magnesium sulfate (42 mg, 0.35 mmol, 3.5 equiv.) were mixed with chloroform (0.2 mL), followed by stirring at room temperature overnight in an argon atmosphere. The reaction liquid was passed through Celite, the obtained filtrate was concentrated, and the residue was purified by silica gel column chromatography (eluate: a mixed solvent of hexane/ethyl acetate), thus yielding white solid amine (3c) (24 mg, yield: 85%).

### Step: 3

A solution of triphosgene (18 mg, 0.06 mmol, 1 equiv.) in dichloromethane (0.3 mL) was cooled to 0°C, and amine (3c) (17 mg, 0.06 mmol, 1 equiv.) and triethylamine (25 µL, 0.18 mmol, 3 equiv.) were added thereto, followed by stirring for 2 hours in an argon atmosphere. While the reaction liquid was maintained at 0°C, dichloromethane (0.6 mL), 5-hydroxy-3-methyl-2-furanone (4a) (21 mg, 0.18 mmol, 3 equiv.), triethylamine (25 µL, 0.18 mmol, 3 equiv.), and N,N-dimethyl-4-aminopyridine (0.7 mg, 0.006 mmol, 10 mol%) were added thereto. The reaction liquid was stirred overnight while its temperature was slowly returned to room temperature. The reaction liquid was quenched with saturated sodium hydrogen carbonate, extracted with dichloromethane (3 mL) (× 3), and dried over anhydrous Na₂SO₄. After the solvent was evaporated, the residue was purified by silica gel column chromatography (eluate: a mixed solvent of hexane/ethyl acetate), thus yielding a white solid target product (12) (compound 27) (21 mg, yield: 82%).
¹H NMR (600 MHz, CDCl₃) rotamer A δ 7.73 (2H, d, J = 9.0 Hz), 7.02 (2H, d, J = 9.0 Hz), 6.88 (1H, s), 6.81 (1H, s), 4.68 (1H, d, J = 9.0 Hz), 4.62 (1H, d, J = 9.0 Hz), 4.09-4.01 (2H, m), 3.57 (1H, dt, J = 12.0, 6.0 Hz), 3.53-3.48 (1H, m), 3.32 (1H, dt, J = 9.6, 6.6 Hz), 3.26-3.18 (1H, m), 2.02 (3H, s), 1.84-1.77 (2H, m), 1.51 (2H, sextet, J = 7.8 Hz), 0.99 (3H, t, J = 7.8 Hz); rotamer B δ 7.76 (2H, d, J = 9.0 Hz), 7.00 (2H, d, J = 9.0 Hz), 6.81 (1H, s), 6.79 (1H, s), 4.72 (2H, s), 4.09-4.01 (2H, m), 3.60 (1H, dt, J = 12.0, 6.0 Hz), 3.53-3.48 (1H, m), 3.26-3.18 (2H, m), 1.96 (3H, s), 1.84-1.77 (2H, m), 1.51 (2H, sextet, J = 7.8 Hz), 0.99 (3H, t, J = 7.8 Hz); ¹³C NMR (151 MHz, CDCl₃) mixture of rotamers δ 171.1, 171.0, 163.6₃, 163.5₈, 150.9, 150.6, 141.8₉, 141.8₆, 134.9, 134.7, 130.0, 129.8, 127.2₃, 127.1₈, 115.4, 115.2, 93.5, 68.5₁, 68.4₈, 62.5, 61.9, 47.2, 46.4, 44.2, 44.0, 31.1, 19.3, 13.9, 10.8, 10.7; IR (film) 2959, 2874, 1778, 1726, 1593, 1422, 1348, 1260, 1157, 1092, 959 cm⁻¹; HRMS (ESI) Calcd for C₁₉H₂₄O₇N₂NaS⁺ ([M+Na]⁺) 447.1196, Found 447.1191.

### Synthesis of Compound 28

Synthesis was performed using appropriate material compounds according to the synthesis method for compound 27.
Compound 28: White solid (16 mg, yield: 45%). ¹H NMR (600 MHz, CDCl₃) rotamer A δ 7.73 (2H, d, J = 9.0 Hz), 7.31 (1H, d, J = 6.0 Hz), 7.02 (2H, d, J = 9.0 Hz), 6.94 (1H, s), 6.35 (1H, d, J = 6.0 Hz), 4.68 (1H, d, J = 9.0 Hz), 4.63 (1H, d, J = 9.0 Hz), 4.09-4.01 (2H, m), 3.57 (1H, dt, J = 12.0, 6.0 Hz), 3.51 (1H, dt, J = 12.0, 6.0 Hz), 3.33 (1H, dt, J = 9.6, 6.0 Hz), 3.29-3.19 (1H, m), 1.80 (2H, quintet, J = 7.2 Hz), 1.51 (2H, sextet, J = 7.2 Hz), 0.99 (3H, t, J = 7.2 Hz); rotamer B δ 7.77 (2H, d, J = 9.0 Hz), 7.24 (1H, d, J = 6.0 Hz), 7.01 (2H, d, J = 9.0 Hz), 6.92 (1H, s), 6.28 (1H, d, J = 6.0 Hz), 4.72 (2H, s), 4.09-4.01 (2H, m), 3.61 (1H, dt, J = 12.0, 6.0 Hz), 3.51 (1H, dt, J = 12.0, 6.0 Hz), 3.29-3.19 (2H, m), 1.80 (2H, quintet, J = 7.2 Hz), 1.51 (2H, sextet, J = 7.2 Hz), 0.99 (3H, t, J = 7.2 Hz); ¹³C NMR (151 MHz, CDCl₃) mixture of rotamers δ 169.6, 169.5, 163.6₄, 163.5₉, 150.6, 150.4, 149.6, 130.0, 129.8, 127.2, 127.1, 125.6, 125.5, 115.4, 115.2, 95.0, 68.5₁, 68.4₇, 62.5, 61.9, 47.2, 46.4, 44.3, 44.1, 31.1, 19.3, 13.9; IR (film) 2959, 2874, 1792, 1728, 1593, 1420, 1346, 1260, 1157, 1086, 1009 cm⁻¹; HRMS (ESI) Calcd for C₁₈H₂₂O₇N₂NaS⁺ ([M+Na]⁺) 433.1040, Found 433.1031.

### Synthesis of 29

Compound 29 was synthesized according to the following synthetic scheme.

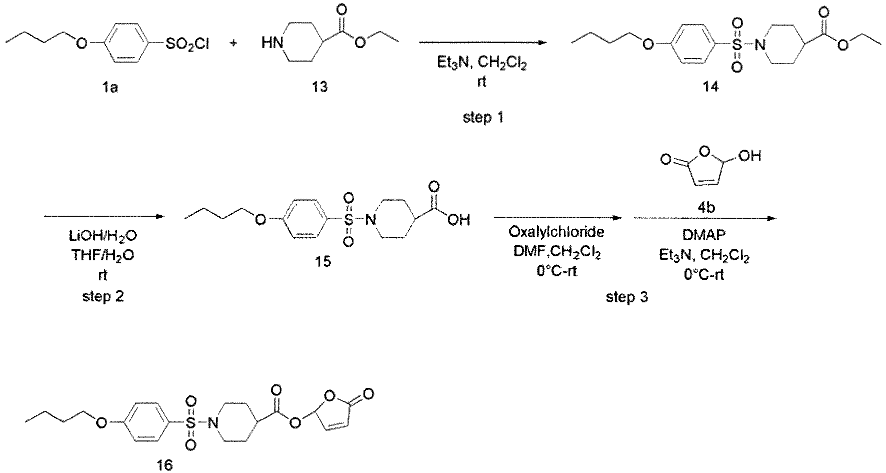

### Step: 1

4-Butoxybenzene-1-sulfonyl chloride (1a) (0.25 g, 1.0 mmol, 1 equiv.) and triethylamine (0.21 mL, 1.5 mmol, 1.5 equiv.) were added to a solution of ethyl 4-piperidinecarboxylate (13) (0.15 mL, 1.0 mmol, 1 equiv.) in dichloromethane (10 mL), and the resulting mixture was stirred at room temperature overnight; thereafter, the mixture was quenched with saturated sodium hydrogen carbonate. The reaction mixture was extracted with dichloromethane (5 mL) (× 3) and dried over anhydrous Na₂SO₄. After the solvent was evaporated, the residue was purified by silica gel column chromatography (eluate: a mixed solvent of hexane/ethyl acetate), thus yielding white solid sulfonamide (14) (0.32 g, yield: 87%).

### Step: 2

In a mixed solution of tetrahydrofuran (1.5 mL) and water (1.5 mL), lithium hydroxide monohydrate (63 mg, 1.5 mmol, 5 equiv.) and sulfonamide (14) (110 mg, 0.3 mmol, 1 equiv.) were stirred at room temperature overnight. The reaction mixture was diluted with water and extracted with diethyl ether (5 mL) (× 3). The aqueous layer was adjusted to pH 2 with potassium hydrogen sulfate, then extracted with ethyl acetate (5 mL) (× 3), dried over anhydrous Na₂SO₄, and concentrated, thus yielding while solid sulfonamide (15) (94 mg, yield: 92%).

### Step: 3

A solution of sulfonamide (15) (34 mg, 0.10 mmol, 1 equiv.) in dichloromethane (1 mL) was cooled to 0°C, oxalylchloride (9.4 µL, 0.11 mmol, 1.1 equiv.) and N,N-dimethylformamide (one drop) were added thereto, and the mixture was stirred for 5 hours in an argon atmosphere while its temperature was slowly returned to room temperature. After the reaction liquid was concentrated, dichloromethane (1 mL), 5-hydroxy-2-furanone (4b) (30 mg, 0.3 mmol, 3 equiv.), triethylamine (42 µL, 0.3 mmol, 3 equiv.), and N,N-dimethyl-4-aminopyridine (1 mg, 0.01 mmol, 10 mol%) were added thereto at 0°C. The reaction liquid was stirred overnight while its temperature was slowly returned to room temperature. The reaction liquid was quenched with saturated sodium hydrogen carbonate, extracted with dichloromethane (3 mL) (× 3), and dried over anhydrous Na₂SO₄. After the solvent was evaporated, the residue was purified by silica gel column chromatography (eluate: a mixed solvent of hexane/ethyl acetate), thus yielding a white solid target product (16) (compound 29). (13 mg, yield: 31%).
¹H NMR (600 MHz, CDCl₃) δ 7.67 (2H, d, J = 9.0 Hz), 7.29 (1H, d, J = 5.4 Hz), 6.98 (2H, d, J = 9.0 Hz), 6.97 (1H, s), 6.31 (1H, d, J = 5.4 Hz), 4.02 (2H, t, J = 7.8 Hz), 3.65-3.59 (2H, m), 2.48 (2H, t, J = 11.4 Hz), 2.39-2.33 (1H, m), 2.00 (2H, t, J = 9.6 Hz), 1.88-1.77 (4H, m), 1.51 (2H, sextet, J = 7.8 Hz), 0.99 (3H, t, J = 7.8 Hz); ¹³C NMR (151 MHz, CDCl₃) δ 172.1, 169.5, 162.9, 149.5, 129.8, 127.4, 125.5, 114.8, 94.0, 68.3, 45.2₈, 45.2₅, 40.0, 31.2, 27.3, 19.3, 13.9, one carbon atom was not found due to overlapping; IR (film) 2959, 2934, 2872, 1792, 1757, 1595, 1497, 1335, 1260, 1155, 1086, 1009, 928 cm⁻¹; HRMS (ESI) Calcd for C₂₀H₂₅O₇NNaS⁺ ([M+Na]⁺) 446.1244, Found 446.1240.

### Synthesis of Compound 30 (Reference)

Compound 30 was synthesized according to the following synthetic scheme.

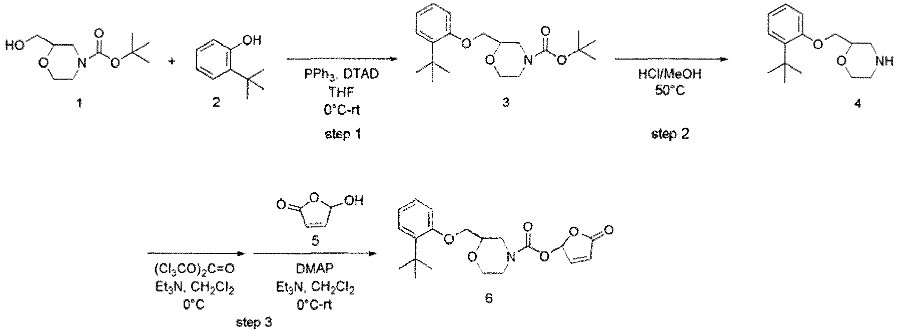

### Step: 1

A solution of (S)-N-Boc-2-hydroxymethylmorpholine (1) (21.7 mg, 0.1 mmol, 1 equiv.), 2-tert-butylphenol (2) (18.4 µL, 0.12 mmol, 1.2 equiv.), and triphenylphosphine (31.5 mg, 0.12 mmol, 1.2 equiv.) in tetrahydrofuran (1 mL) was cooled to 0°C, and di-tert-butylazodicarboxylate (27.6 mg, 0.12 mmol, 1.2 equiv.) was added thereto. Then, the reaction liquid was stirred overnight in an argon atmosphere while its temperature was slowly returned to room temperature. The reaction liquid diluted with water was extracted with ethyl acetate (5 mL) (× 3), and the organic layer was washed with an aqueous saturated sodium chloride solution and dried over anhydrous Na₂SO₄. After the solvent was evaporated, the residue was purified by silica gel column chromatography (eluate: a mixed solvent of hexane/ethyl acetate), thus yielding amide (3) in the form of yellow oil (24.4 mg, yield: 70%).

### Step: 2

Amide (3) (24.4 mg, 0.07 mmol, 1 equiv.) and a 6N hydrochloric acid/methanol solution (0.35 mL) were stirred at 50°C overnight. The solvent was evaporated, and the residue was dissolved in water (5 mL) and washed with diethyl ether. The obtained aqueous layer was adjusted to a pH of 9 with sodium hydrogen carbonate, and then extracted with ethyl acetate (5 mL) (× 3). The organic layer was dried over anhydrous Na₂SO₄. After the solvent was evaporated, the residue was purified by silica gel column chromatography (eluate: mixed solvent of chloroform/methanol), thus yielding amine (4) in the form of yellow oil (8.3 mg, yield: 47%).

### Step: 3

A solution of triphosgene (9.8 mg, 0.033 mmol, 1 equiv.) in dichloromethane (1.0 mL) was cooled to 0°C, and amine (4) (8.3 mg, 0.033 mmol, 1 equiv.) and triethylamine (14 µL, 0.099 mmol, 3 equiv.) were added thereto, followed by stirring for 2 hours in an argon atmosphere. While the reaction liquid was maintained at 0°C, dichloromethane (3.6 mL), 5-hydroxy-5H-2-furanone (5) (10 mg, 0.099 mmol, 3 equiv.), triethylamine (14 µL, 0.099 mmol, 3 equiv.), and N,N-dimethyl-4-aminopyridine (0.4 mg, 0.0033 mmol, and 10 mol%) were added thereto. The reaction liquid was stirred overnight while its temperature was slowly returned to room temperature. A saturated aqueous sodium hydrogen carbonate solution was added to the reaction liquid, extraction was performed with dichloromethane (3 mL) (× 3), and the organic layer was dried over anhydrous Na₂SO₄. After the solvent was evaporated, the residue was purified by silica gel column chromatography (eluate: a mixed solvent of hexane/ethyl acetate), thus yielding a target product (6) in the form of colorless oil (compound 30). (10.2 mg, yield: 82%).
¹H NMR (600 MHz, CDCl₃) mixture of diastereomers and rotamers δ 7.36-7.27 (2H, m), 7.17 (1H, t, J = 8.2 Hz), 7.06 (1H, br), 6.92 (1H, t, J= 8.2 Hz), 6.86-6.80 (1H, m), 6.32 (1H, d, J= 6.0 Hz), 4.33-4.17 (1H, m), 4.13-3.80 (5H, m), 3.73-3.57 (1H, m), 3.20-3.08 (1H, m), 3.08-3.00 (1H, m), 1.41-1.33 (9H, m); ¹³C NMR (151 MHz, DMSO-d6) mixture of diastereomers and rotamers δ 170.9, 157.3, 152.8, 152.5, 137.7, 127.7, 126.8, 124.7, 121.0, 112.9, 112.7, 95.9, 95.7, 74.0, 73.8, 68.6, 68.1, 66.2, 66.0, 46.8, 46.1, 44.3, 43.9, 34.9, 30.2; IR (film) 2951, 2926, 2864, 1794, 1719, 1491, 1443, 1233, 1126, 1092, 1015, 978 cm⁻¹; HRMS (ESI) Calcd for C₂₀H₂₅O₆NNa⁺ ([M+Na]⁺) 398.1574, Found 398.1573.

### Reference Example 1: Synthesis of Comparison Compound

Compound 2 disclosed in PTL 1 was synthesized as Comparison Compound 1 using the method disclosed in PTL 1. The structural formula of Comparison Compound 1 is as follows.

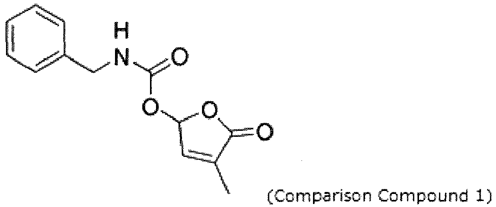

### Reference Example 2: Synthesis of Probe for Evaluation of Affinity for Strigolactone Receptor

A probe (YLG) for evaluation of affinity for the strigolactone receptor disclosed in PTL 2 was synthesized using the method disclosed in PTL 2. The structural formula of YLG is as follows.

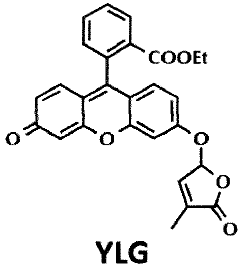

### Example 2: Evaluation of Parasitic Plant Germination Regulation Activity

A parasitic plant germination induction test was conducted using the compounds synthesized in Example 1 as test compounds. The seeds of a parasitic plant (*Striga hermonthica, Orobanche minor,* or *Phelipanche aegyptiaca*) were suspended in sterilized ultrapure water, and this was dispensed in an amount of 100 µL each (containing about 20 seeds) into a 96-well plate. The test compounds or control compounds (Comparison Compound 1 or synthetic Strigolactone (GR²⁴: Comparison Compound 2)) dissolved in DMSO were individually added thereto to a final concentration of these compounds of 10 nM to 1 fM (1 × 10⁻⁸ to 1 × 10⁻¹⁵ M), and allowed to stand in the dark for 2 days. The presence or absence of germination was observed using a microscope, and the germination rate (i.e., number of germinated seeds/total number of seeds) was measured. The higher the germination rate in the parasitic plant germination induction test, or the higher the germination rate at lower concentration, the higher the *Striga* germination inducing activity.

Tables 1 to 6 show the results. Tables 1 to 4 show the results of comparison using *Striga hermonthica* seeds. Table 5 shows the results of comparison using the seeds of *Orobanche minor.* Table 6 shows the results of comparison using the seeds of *Phelipanche aegyptiaca.* In Tables 1 to 6, the concentrations (10 nM, 1 nM, and 0.1 nM) represent compound concentrations. In Tables 1-6, the evaluation criteria for the compounds are as follows:
++: germination rate of 20% or more
+: germination rate of 5% or more and less than 20%
-: germination rate of less than 5%

**Table 1**

| | Structural formula | Germination rate 10 nM | Germination rate 1 nM | Germination rate 0.1 nM |
|---|---|---|---|---|
| Compound 1 | 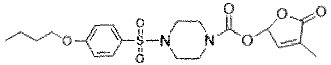 | ++ | ++ | ++ |
| Compound 2 | 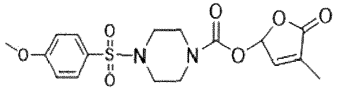 | ++ | ++ | ++ |
| Compound 3 | 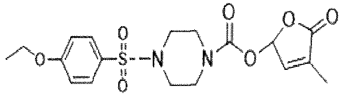 | ++ | ++ | ++ |
| Compound 4 | 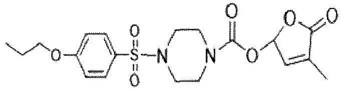 | ++ | ++ | ++ |
| Compound 5 | 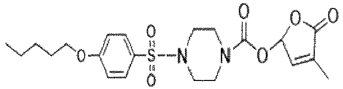 | ++ | ++ | ++ |
| Compound 6 | 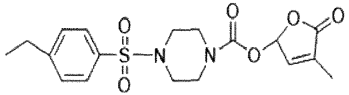 | ++ | ++ | ++ |
| Compound 7 | 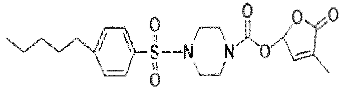 | ++ | ++ | ++ |
| Compound 8 | 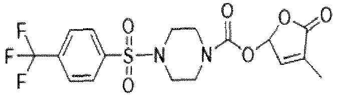 | ++ | ++ | ++ |

**Table 2**

| | Structural formula | Germination rate 10 nM | Germination rate 1 nM | Germination rate 0.1 nM |
|---|---|---|---|---|
| Compound 9 | 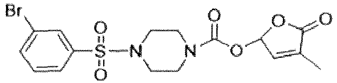 | ++ | ++ | ++ |
| Compound 10 | 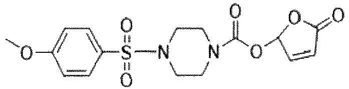 | ++ | ++ | - |
| Compound 11 | 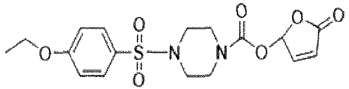 | ++ | ++ | + |
| Compound 12 | 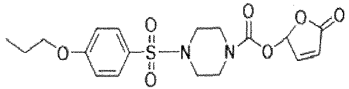 | ++ | ++ | + |
| Compound 13 | 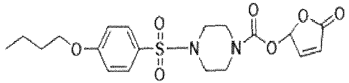 | ++ | ++ | + |
| Compound 14 | 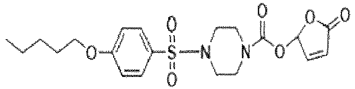 | ++ | ++ | - |
| Compound 15 | 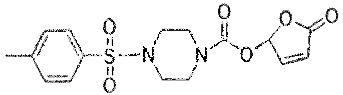 | ++ | + | - |
| Compound 16 | 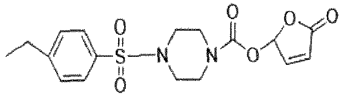 | ++ | ++ | |

**Table 3**

| | Structural formula | Germination rate 10 nM | Germination rate 1 nM | Germination rate 0.1 nM |
|---|---|---|---|---|
| Compound 17 | 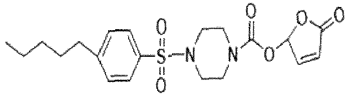 | ++ | + | + |
| Compound 18 | 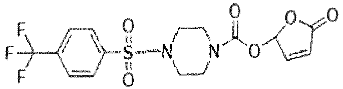 | ++ | + | - |
| Compound 19 | 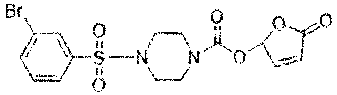 | ++ | ++ | - |
| Compound 20 | 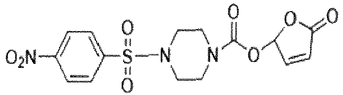 | ++ | ++ | - |
| Compound 21 | 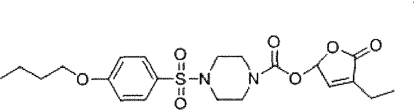 | ++ | ++ | ++ |
| Compound 22 | 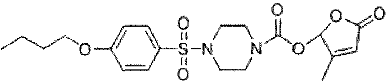 | + | - | - |
| Compound 23 | 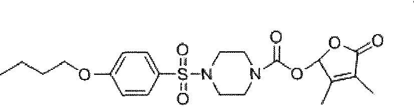 | + | - | - |
| Compound 24 | 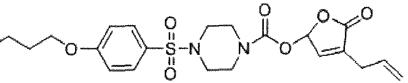 | ++ | ++ | ++ |

**Table 4**

| | Structural formula | Germination rate 10 nM | Germination rate 1 nM | Germination rate 0.1 nM |
|---|---|---|---|---|
| Compound 25 | 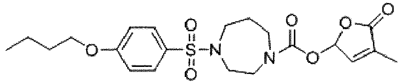 | ++ | ++ | |
| Compound 26 | 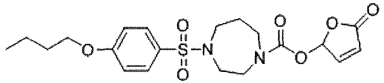 | ++ | ++ | ++ |
| Compound 27 | 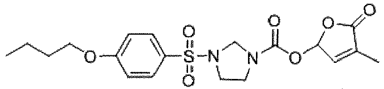 | ++ | ++ | + |
| Compound 28 | 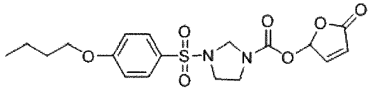 | ++ | - | - |
| Compound 29 | 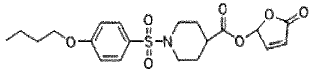 | + | - | - |
| Reference Compound 30 | 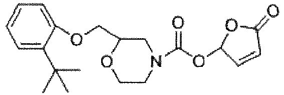 | ++ | ++ | - |
| Comparison Compound 1 | 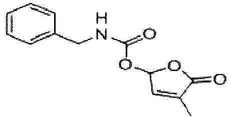 | - | - | - |
| Comparison Compound 2 | 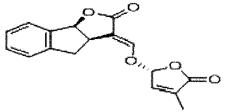 | ++ | ++ | - |

**Table 5**

| | Structural formula | Germination rate 10 nM | Germination rate 1 nM | Germination rate 0.1 nM |
|---|---|---|---|---|
| Compound 1 | 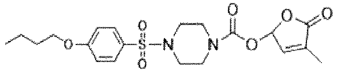 | ++ | ++ | - |
| Comparison Compound 2 | 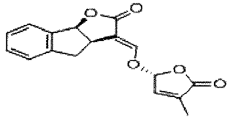 | ++ | + | - |

**Table 6**

| | Structural formula | Germination rate 10 nM | Germination rate 1 nM |
|---|---|---|---|
| Compound 1 | 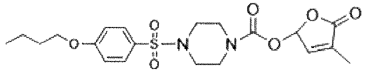 | + | - |
| Comparison Compound 2 | 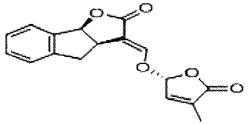 | ++ | - |

As shown in Tables 1 to 4, compounds 1 to 30 all showed higher parasitic plant germination regulation activity than Comparison Compound 1. Further, compounds 1 to 30 generally showed parasitic plant germination regulation activity equivalent to or greater than that of strigolactones.

### Example 3: Evaluation of Affinity for Receptor

PTL 1 reports that the affinity of a test compound for the strigolactone receptor can be evaluated by reacting strigolactone with a probe for evaluation of affinity for a strigolactone receptor (e.g., YLG) in the presence of the test compound, and measuring the fluorescence emitted from the decomposition product of YLG. Therefore, the compounds synthesized in Example 1 were used as the test compounds, and the affinity for the strigolactone receptor was evaluated by a similar method to the above. The details are as follows.

An *Arabidopsis thaliana* strigolactone receptor (AtD14) was produced in accordance with a usual method. More specifically, cDNA of the receptor was obtained by RT-PCR, and expression and purification were carried out in *E*. *coli.* Additionally, *Striga* strigolactone receptors were produced by the method disclosed in PTL 1 (ShHTL1: amino acid SEQ ID NO: 1, ShHTL2: amino acid SEQ ID NO: 2, ShHTL3: amino acid SEQ ID NO: 3, ShHTL4: amino acid SEQ ID NO: 4, ShHTL5: amino acid SEQ ID NO: 5, ShHTLS: amino acid SEQ ID NO: 6, ShHTL7: amino acid SEQ ID NO: 7, ShHTLS: amino acid SEQ ID NO: 8, ShHTL9: amino acid SEQ ID NO: 9, ShHTL10: amino acid SEQ ID NO: 10, and ShHTL11: amino acid SEQ ID NO: 11.)

One hundred microliters of a reaction solution containing each of the obtained strigolactone receptors (1 pg each), YLG (final concentration: 1 pM), and each of the test compounds (final concentration: 0.01 to 10 µM) or the control compound (5-deoxystrigol, final concentration: 0.01 to 10 µM) as a competitive compound (100 mM HEPES, 150 mM NaCl, pH: 7.0, 0.1% DMSO) was prepared and reacted in a 96 black-well plate (Greiner). Sixty after the initiation of the reaction, fluorescence was detected at an excitation wavelength of 480 nm and a detection wavelength of 520 nm using a fluorescence detector (SpectraMax i3, manufactured by Molecular Devices), and the fluorescence intensity was measured. Based on the measurement results of fluorescence intensity, a Lineweaver-Burk plot was created to calculate the Kₘ values, and the IC₅₀ values of each of the test compounds and the control compound, which are competitive compounds.

Table 7 shows the results. In Table 7, HTL1 to HTL11 refer to strigolactone receptors (SEQ ID NOs: 1 to 11), and AtD14 refer to the *Arabidopsis thaliana* strigolactone receptor.

**Table 7**

| Strigolactone receptor | IC50 values of test compounds (uM) | | | |
|---|---|---|---|---|
| | Compound 1 | Compound 13 | Comparison Compound 1 | 5-Ddeoxystriqol |
| HTL2 | >10 | >10 | >10 | 8.9 |
| HTL3 | >10 | 2 | >10 | 9.3 |
| HTL4 | >10 | >10 | >10 | 0.3 |
| HTL5 | >10 | >10 | >10 | 3.6 |
| HTL6 | >10 | >10 | >10 | 0.19 |
| HTL7 | 0.31 | 0.75 | >10 | 0.12 |
| HTL8 | 1.1 | >10 | >10 | 0.36 |
| HTL9 | >10 | >10 | >10 | 1.2 |
| HTL10 | >10 | >10 | >10 | 0.62 |
| HTL11 | 1.8 | >10 | 0.66 | 1.6 |
| AtD14 | >10 | >10 | NT | 0.44 |

| | | | | |
|---|---|---|---|---|
| NT: Not tested | | | | |

Table 7 suggests that the test compounds had affinity for the *Striga* strigolactone receptors, in particular ShHTL7, which is considered to be the most important receptor among these. It was also found that the test compounds did not have affinity for the *Arabidopsis thaliana* strigolactone receptor, and had affinity specific for the *Striga* strigolactone receptors. This suggests that the test compounds had a lower impact on plants other than parasitic plants (e.g., crops).

## Claims

1. A compound represented by Formula (1), an agriculturally acceptable salt thereof, a hydrate thereof, or a solvate thereof: 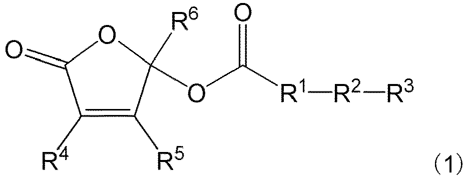 wherein
R¹ is a divalent group represented by Formula (Y): 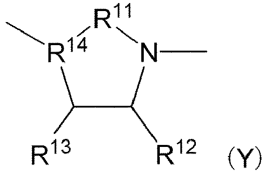
wherein
R¹¹ represents -(CHR^{11a}-)ₙ, wherein n represents an integer of 1 to 3, R^{11a}s are identical or different, and each represents hydrogen or alkyl, and when n is 2 or 3, two R^{11a}s, taken together with the adjacent carbon atoms, may form a ring,
R¹² and R¹³ are identical or different, and each represents hydrogen or alkyl, and R¹² and R¹³, taken together with the adjacent carbon atoms, may form a ring, and
R¹⁴ represents carbon or nitrogen; or
R¹ is a divalent group represented by Formula (Y'): 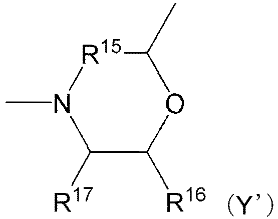
wherein
R¹⁵ represents a single bond or -(CHR^{15a}-)ₘ, wherein m represents 1 or 2, R^{15a}s are identical or different, and each represents hydrogen or alkyl, and when m is 2, two adjacent R^{15a}s, taken together with the adjacent carbon atoms, may form a ring, and
R¹⁶ and R¹⁷ are identical or different, and each represents hydrogen or alkyl, and R¹⁶ and R¹⁷, taken together with the adjacent carbon atoms, may form a ring,
R² represents -S(O)₂-,
R³ represents optionally substituted phenyl,
R⁴, R⁵, and R⁶ are identical or different, and each represents hydrogen, halogen, optionally substituted alkyl, optionally substituted alkenyl, or optionally substituted alkynyl.

2. The compound, an agriculturally acceptable salt thereof, a hydrate thereof, or a solvate thereof according to claim 1, wherein the compound represented by Formula (1) is a compound represented by Formula (1C): 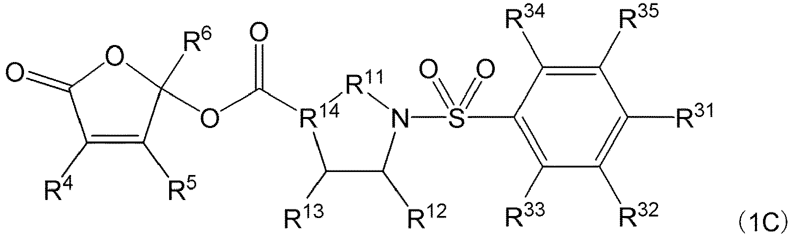
wherein R¹¹ represents - (CHR^{11a}-)ₙ, wherein n represents an integer of 1 to 3, R^{11a}s are identical or different, and each represents hydrogen or alkyl, and when n is 2 or 3, two R^{11a}s, taken together with the adjacent carbon atoms, may form a ring,
R¹² and R¹³ are identical or different, and each represents hydrogen or alkyl, and R¹² and R¹³, taken together with the adjacent carbon atoms, may form a ring,
R¹⁴ represents carbon or nitrogen,
R³¹, R³², R³³, R³⁴, and R³⁵ are identical or different, and each represents hydrogen, halogen, optionally substituted alkyl, optionally substituted alkoxy, optionally substituted alkylthio, optionally substituted alkenyl, optionally substituted alkynyl, optionally substituted amino, nitro, hydroxyl, cyano, -C(=O)R³⁰¹, -C(=O)OR³⁰², -C (=O)NR³⁰³R³⁰⁴, - S (=O)₁₋₂R³⁰⁵, or -S (=O)₁₋₂NR³⁰⁶R³⁰⁷, wherein R³⁰¹, R³⁰², R³⁰³, R³⁰⁴, R³⁰⁵, R³⁰⁶, and R³⁰⁷ each represent hydrogen, optionally substituted alkyl, or optionally substituted phenyl; and
R⁴, R⁵, and R⁶ are identical or different, and each represents hydrogen, halogen, optionally substituted alkyl, optionally substituted alkenyl, or optionally substituted alkynyl.

3. The compound, an agriculturally acceptable salt thereof, a hydrate thereof, or a solvate thereof according to claim 2, wherein R⁴ is optionally substituted alkyl, optionally substituted alkenyl, or optionally substituted alkynyl, and both R⁵ and R⁶ are hydrogen atoms.

4. A parasitic plant germination regulator comprising the compound according to any one of claims 1 to 3, an agriculturally acceptable salt thereof, a hydrate thereof, or a solvate thereof.

5. A method for regulating parasitic plant germination, comprising applying the compound according to any one of claims 1 to 3, an agriculturally acceptable salt thereof, a hydrate thereof, or a solvate thereof to soil containing parasitic plant seeds.

6. The method according to claim 5, wherein parasitic plant germination is induced.

7. The method according to claim 5 or 6, wherein the parasitic plant is a plant of the genus *Striga* or the genus *Orobanche.*

8. Use of the compound according to any one of claims 1 to 3, an agriculturally acceptable salt thereof, a hydrate thereof, or a solvate thereof, for regulating parasitic plant germination, comprising applying the the compound according to any one of claims 1 to 3, an agriculturally acceptable salt thereof, a hydrate thereof, or a solvate thereof, to soil containing parasitic plant seeds.

9. The use according to claim 8, wherein the compound according to any one of claims 1 to 3, an agriculturally acceptable salt thereof, a hydrate thereof, or a solvate thereof, is used for inducing parasitic plant germination.

10. The use according to claim 8 or 9, wherein the parasitic plant is a plant of the genus *Striga* or the genus *Orobanche.*

## Patentansprüche

1. Eine Verbindung, dargestellt durch Formel (1), ein landwirtschaftlich verträgliches Salz davon, ein Hydrat davon oder ein Solvat davon: 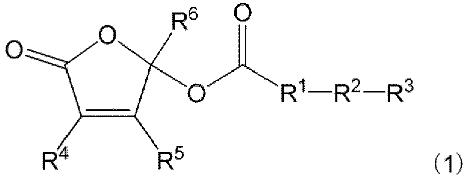 wobei
R¹ eine zweiwertige Gruppe ist, dargestellt durch Formel (Y): 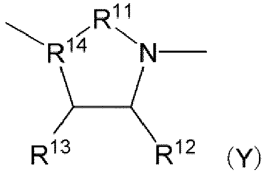
wobei
R¹¹ -(CHR^{11a}-)ₙ darstellt, wobei n eine ganze Zahl von 1 bis 3 darstellt, R^{11a} gleich oder verschieden sind und jeweils Wasserstoff oder Alkyl darstellen und, wenn n gleich 2 oder 3 ist, zwei R^{11a}, zusammen mit den benachbarten Kohlenstoffatomen, einen Ring bilden können,
R¹² und R¹³ gleich oder verschieden sind und jeweils Wasserstoff oder Alkyl darstellen und R¹² und R¹³, zusammen mit den benachbarten Kohlenstoffatomen, einen Ring bilden können, und
R¹⁴ Kohlenstoff oder Stickstoff darstellt; oder
R¹ eine zweiwertige Gruppe ist, dargestellt durch die Formel (Y'): 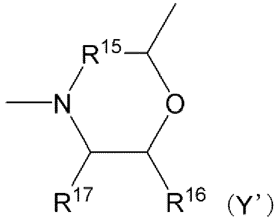
wobei
R¹⁵ eine Einfachbindung oder -(CHR^{15a}-)ₘ darstellt, wobei m 1 oder 2 darstellt, R^{15a} gleich oder verschieden sind, und jeweils Wasserstoff oder Alkyl darstellen und, wenn m gleich 2 ist, zwei benachbarte R^{15a}, zusammen mit den benachbarten Kohlenstoffatomen, einen Ring bilden können, und
R¹⁶ und R¹⁷ gleich oder verschieden sind und jeweils Wasserstoff oder Alkyl darstellen und R¹⁶ und R¹⁷, zusammen mit den benachbarten Kohlenstoffatomen, einen Ring bilden können,
R² -S(O)₂- darstellt,
R³ gegebenenfalls substituiertes Phenyl darstellt,
R⁴, R⁵ und R⁶ gleich oder verschieden sind und jeweils Wasserstoff, Halogen, gegebenenfalls substituiertes Alkyl, gegebenenfalls substituiertes Alkenyl oder gegebenenfalls substituiertes Alkinyl darstellen.

2. Die Verbindung, ein landwirtschaftlich verträgliches Salz davon, ein Hydrat davon oder ein Solvat davon gemäß Anspruch 1, wobei die Verbindung, dargestellt durch Formel (1), eine Verbindung, dargestellt durch Formel (1C), ist: 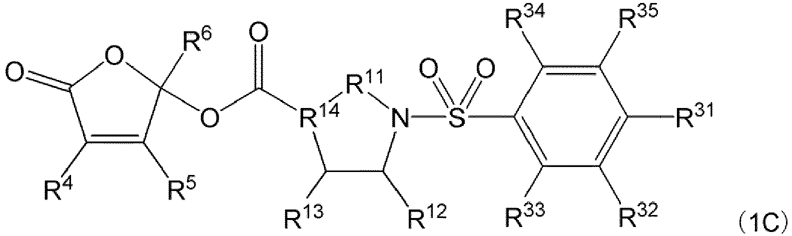
wobei R¹¹ -(CHR^{11a}-)ₙ darstellt, wobei n eine ganze Zahl von 1 bis 3 darstellt, R^{11a} gleich oder verschieden sind, und jeweils Wasserstoff oder Alkyl darstellen und, wenn n gleich 2 oder 3 ist, zwei R^{11a}, zusammen mit den benachbarten Kohlenstoffatomen, einen Ring bilden können,
R¹² und R¹³ gleich oder verschieden sind und jeweils Wasserstoff oder Alkyl darstellen und R¹² und R¹³, zusammen mit den benachbarten Kohlenstoffatomen, einen Ring bilden können,
R¹⁴ Kohlenstoff oder Stickstoff darstellt,
R³¹, R³², R³³, R³⁴ und R³⁵ gleich oder verschieden sind und jeweils Wasserstoff, Halogen, gegebenenfalls substituiertes Alkyl, gegebenenfalls substituiertes Alkoxy, gegebenenfalls substituiertes Alkylthio, gegebenenfalls substituiertes Alkenyl, gegebenenfalls substituiertes Alkinyl, gegebenenfalls substituiertes Amino, Nitro, Hydroxyl, Cyano, -C(=O)R³⁰¹, -C(=O)OR³⁰², -C(=O)NR³⁰³R³⁰⁴, -S(=O)₁₋₂R³⁰⁵ oder -S(=O)₁₋₂NR³⁰⁶R³⁰⁷ darstellen, wobei R³⁰¹, R³⁰², R³⁰³, R³⁰⁴, R³⁰⁵, R³⁰⁶ und R³⁰⁷ jeweils Wasserstoff, gegebenenfalls substituiertes Alkyl, oder gegebenenfalls substituiertes Phenyl darstellen; und
R⁴, R⁵, und R⁶ gleich oder verschieden sind und jeweils Wasserstoff, Halogen, gegebenenfalls substituiertes Alkyl, gegebenenfalls substituiertes Alkenyl oder gegebenenfalls substituiertes Alkinyl darstellen.

3. Die Verbindung, ein landwirtschaftlich verträgliches Salz davon, ein Hydrat davon oder ein Solvat davon gemäß Anspruch 2, wobei R⁴ gegebenenfalls substituiertes Alkyl, gegebenenfalls substituiertes Alkenyl oder gegebenenfalls substituiertes Alkinyl ist und beide Reste R⁵ und R⁶ Wasserstoffatome sind.

4. Ein Keimungsregulator für parasitische Pflanzen, umfassend die Verbindung gemäß einem der Ansprüche 1 bis 3, ein landwirtschaftlich verträgliches Salz davon, ein Hydrat davon oder ein Solvat davon.

5. Ein Verfahren zum Regulieren der Keimung von parasitischen Pflanzen, umfassend das Aufbringen der Verbindung gemäß einem der Ansprüche 1 bis 3, eines landwirtschaftlich verträgliches Salzes davon, eines Hydrats davon oder eines Solvats davon auf Boden, der Samen von parasitischen Pflanzen enthält.

6. Das Verfahren gemäß Anspruch 5, wobei die Keimung der parasitischen Pflanze angeregt wird.

7. Das Verfahren gemäß Anspruch 5 oder 6, wobei die parasitische Pflanze eine Pflanze der Gattung *Striga* oder der Gattung *Orobanche* ist.

8. Verwendung der Verbindung gemäß einem der Ansprüche 1 bis 3, eines landwirtschaftlich verträglichen Salzes davon, eines Hydrats davon oder eines Solvats davon zum Regulieren der Keimung von parasitischen Pflanzen, umfassend das Aufbringen der Verbindung gemäß einem der Ansprüche 1 bis 3, eines landwirtschaftlich verträglichen Salzes davon, eines Hydrats davon oder eines Solvats davon auf Boden, der Samen von parasitischen Pflanzen enthält.

9. Die Verwendung gemäß Anspruch 8, wobei die Verbindung gemäß einem der Ansprüche 1 bis 3, ein landwirtschaftlich verträgliches Salz davon, ein Hydrat davon oder ein Solvat davon verwendet wird, um die Keimung der parasitischen Pflanze anzuregen.

10. Die Verwendung gemäß Anspruch 8 oder 9, wobei die parasitische Pflanze eine Pflanze der Gattung *Striga* oder der Gattung *Orobanche* ist.

## Revendications

1. Composé représenté par la formule (1), sel acceptable du point de vue agricole de celui-ci, hydrate de celui-ci ou solvate de celui-ci : 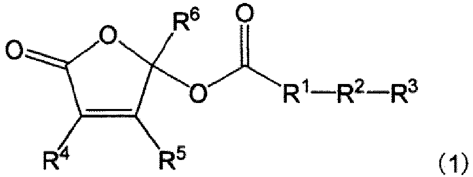 où
R¹ est un groupe divalent représenté par la formule (Y) : 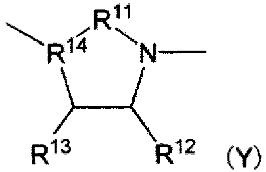
où
R¹¹ représente -(CHR^{11a}-)ₙ, où n représente un nombre entier de 1 à 3, les R^{11a} sont identiques ou différents et représentent chacun un hydrogène ou un alkyle, et lorsque n est 2 ou 3, deux R^{11a}, pris ensemble avec les atomes de carbone adjacents, peuvent former un cycle,
R¹² et R¹³ sont identiques ou différents, et représentent chacun un hydrogène ou un alkyle, et R¹² et R¹³, pris ensemble avec les atomes de carbone adjacents, peuvent former un cycle, et
R¹⁴ représente un carbone ou un azote ; ou
R¹ est un groupe divalent représenté par la formule (Y') : 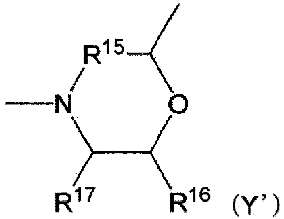
où
R¹⁵ représente une liaison simple ou -(CHR^{15a}-)ₘ, où m représente 1 ou 2, les R^{15a} sont identiques ou différents et représentent chacun un hydrogène ou un alkyle, et lorsque m est 2, deux R^{15a} adjacents, pris ensemble avec les atomes de carbone adjacents, peuvent former un cycle, et
R¹⁶ et R¹⁷ sont identiques ou différents et représentent chacun un hydrogène ou un alkyle, et R¹⁶ et R¹⁷, pris ensemble avec les atomes de carbone adjacents, peuvent former un cycle,
R² représente -S(O)₂-,
R³ représente un phényle éventuellement substitué,
R⁴, R⁵ et R⁶ sont identiques ou différents et représentent chacun un hydrogène, un halogène, un alkyle éventuellement substitué, un alcényle éventuellement substitué ou un alcynyle éventuellement substitué.

2. Composé, sel acceptable du point de vue agricole de celui-ci, hydrate de celui-ci ou solvate de celui-ci selon la revendication 1, où le composé représenté par la formule (1) est un composé représenté par la Formule (1C) : 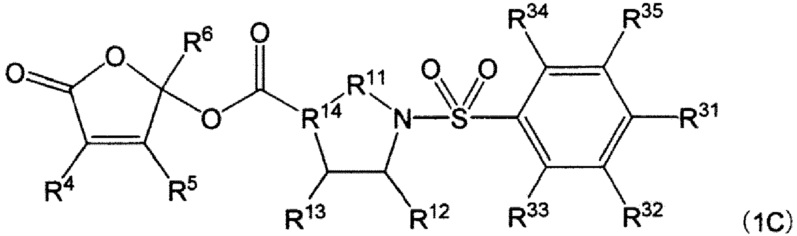
où R¹¹ représente -(CHR^{11a}-)ₙ, où n représente un nombre entier de 1 à 3, les R^{11a} sont identiques ou différents et représentent chacun un hydrogène ou un alkyle, et lorsque n est 2 ou 3, deux R^{11a}, pris ensemble avec les atomes de carbone adjacents, peuvent former un cycle,
R¹² et R¹³ sont identiques ou différents et représentent chacun un hydrogène ou un alkyle, et R¹² et R¹³, pris ensemble avec les atomes de carbone adjacents, peuvent former un cycle,
R¹⁴ représente un carbone ou un azote,
R³¹, R³², R³³, R³⁴ et R³⁵ sont identiques ou différents et représentent chacun un hydrogène, un halogène, un alkyle éventuellement substitué, un alcoxy éventuellement substitué, un alkylthio éventuellement substitué, un alcényle éventuellement substitué, un alcynyle éventuellement substitué, un amino éventuellement substitué, un nitro, un hydroxyle, un cyano, -C(=O)R³⁰¹, -C(=O)OR³⁰², -C(=O)NR³⁰³R³⁰⁴, -S(=O)₁₋₂R³⁰⁵ ou -S(=O)₁₋₂NR³⁰⁶R³⁰⁷, où R³⁰¹, R³⁰², R³⁰³, R³⁰⁴, R³⁰⁵, R³⁰⁶ et R³⁰⁷ représentent chacun un hydrogène, un alkyle éventuellement substitué ou un phényle éventuellement substitué ; et
R⁴, R⁵ et R⁶ sont identiques ou différents et représentent chacun un hydrogène, un halogène, un alkyle éventuellement substitué, un alcényle éventuellement substitué ou un alcynyle éventuellement substitué.

3. Composé, sel acceptable du point de vue agricole de celui-ci, hydrate de celui-ci ou solvate de celui-ci selon la revendication 2, où R⁴ est un alkyle éventuellement substitué, un alcényle éventuellement substitué ou un alcynyle éventuellement substitué, et R⁵ et R⁶ sont tous deux des atomes d'hydrogène.

4. Régulateur de germination de plantes parasites comprenant le composé selon l'une quelconque des revendications 1 à 3, un sel acceptable du point de vue agricole de celui-ci, un hydrate de celui-ci ou un solvate de celui-ci.

5. Procédé pour réguler la germination de plantes parasites comprenant l'application du composé selon l'une quelconque des revendications 1 à 3, d'un sel acceptable du point de vue agricole de celui-ci, d'un hydrate de celui-ci ou d'un solvate de celui-ci à un sol contenant des graines de plantes parasites.

6. Procédé selon la revendication 5, où la germination de plantes parasites est induite.

7. Procédé selon la revendication 5 ou 6, où la plante parasite est une plante du genre *Striga* ou du genre *Orobanche.*

8. Utilisation du composé selon l'une quelconque des revendications 1 à 3, d'un sel acceptable du point de vue agricole de celui-ci, d'un hydrate de celui-ci ou d'un solvate de celui-ci pour réguler la germination de plantes parasites, comprenant l'application du composé selon l'une quelconque des revendications 1 à 3, d'un sel acceptable du point de vue agricole de celui-ci, d'un hydrate de celui-ci ou d'un solvate de celui-ci à un sol contenant des graines de plantes parasites.

9. Utilisation selon la revendication 8, où le composé selon l'une quelconque des revendications 1 à 3, un sel acceptable du point de vue agricole de celui-ci, un hydrate de celui-ci ou un solvate de celui-ci est utilisé pour induire la germination de plantes parasites.

10. Utilisation selon la revendication 8 ou 9, où la plante parasite est une plante du genre *Striga* ou du genre *Orobanche.*
